# EUROPEAN PATENT SPECIFICATION

(11) **EP 2 595 646 B1**
(45) Date of publication and mention of the grant of the patent: **01.07.2015**
(21) Application number: 11734137.0
(22) Date of filing: 21.07.2011
(51) Int. Cl.: A61K 38/17, A61P 25/00, A61P 35/00

(54) **BHLH PROTEINS AND THEIR USE AS DRUGS**
BHLH-PROTEINE UND IHRE VERWENDUNG ALS ARZNEIMITTEL
PROTEINES BHLH ET LEUR UTILISATION EN TANT QUE MEDICAMENTS

(30) Priority: 21.07.2010 EP 10305804
(43) Date of publication of application: 29.05.2013
(73) Proprietor: Université de Montpellier, 34090 Montpellier (FR); INSERM (Institut National de la Santé et de la Recherche Médicale), 75654 Paris Cedex 13 (FR); Université Pierre et Marie Curie (Paris 6), 75005 Paris (FR); Centre National de la Recherche Scientifique (CNRS), 75016 Paris (FR)
(72) Inventor: HUGNOT, Jean-Philippe, F-34000 Montpellier (FR); GUICHET, Pierre-Olivier, F-34090 Montpellier (FR); SERGUERA, Che, F-75010 Paris (FR); MALLET, Jacques, F-75013 Paris (FR)
(74) Representative: Monni, Richard
(86) International application number: PCT/EP2011/062581
(87) International publication number: WO 2012/010675

(56) References cited:
- WO-A1-01/26643
- WO-A1-2004/016779
- WO-A2-98/13491
- US-A1- 2004 152 168
- US-B1- 6 444 463
- HOFSTETTER CHRISTOPH P ET AL: "Allodynia limits the usefulness of intraspinal neural stem cell grafts; directed differentiation improves outcome.", NATURE NEUROSCIENCE MAR 2005 LNKD- PUBMED:15711542, vol. 8, no. 3, March 2005 (2005-03), pages 346-353, XP009139677, ISSN: 1097-6256
- TABU KOUICHI ET AL: "A novel function of OLIG2 to suppress human glial tumor cell growth via p27Kip1 transactivation.", JOURNAL OF CELL SCIENCE 1 APR 2006 LNKD- PUBMED:16554441, vol. 119, no. Pt 7, 1 April 2006 (2006-04-01), pages 1433-1441, XP002603884, ISSN: 0021-9533

### SEQUENCE LISTING

<110> UNIVERSITE MONTPELLIER 2 SCIENCES ET TECHNIQUES Institut National de la Santé et de la Recherche Médicale (INSERM) Université Pierre et Marie Curie (Paris 6) HUGNOT, Jean Philippe GUICHET, Pierre-Olivier SERGUERA, Che MALLET, Jacques
<120> bHLH PRTOTEINS AND THEIR USE AS DRUGS
<130> WOB 10 BI MTP NGN2
<150> EP 10305804.6
   <151> 2010-07-21
<160> 12
<170> BiSSAP 1.0
<210> 1
   <211> 272
   <212> PRT
   <213> Homo sapiens
<220>
   <221> SOURCE
   <222> 1..272
   <223> /mol_type="protein" /organism="Homo sapiens"
<400> 1
   Met Phe Val Lys Ser Glu Thr Leu Glu Leu Lys Glu Glu Glu Asp Val
   1               5                   10                  15
   Leu Val Leu Leu Gly Ser Ala Ser Pro Ala Leu Ala Ala Leu Thr Pro
               20                  25                  30
   Leu Ser Ser Ser Ala Asp Glu Glu Glu Glu Glu Glu Pro Gly Ala Ser
           35                  40                  45
   Gly Gly Ala Arg Arg Gln Arg Gly Ala Glu Ala Gly Gln Gly Ala Arg
       50                  55                  60
   Gly Gly Val Ala Ala Gly Ala Glu Gly Cys Arg Pro Ala Arg Leu Leu
   65                  70                  75                  80
   Gly Leu Val His Asp Cys Lys Arg Arg Pro Ser Arg Ala Arg Ala Val
                   85                  90                  95
   Ser Arg Gly Ala Lys Thr Ala Glu Thr Val Gln Arg Ile Lys Lys Thr
               100                 105                 110
   Arg Arg Leu Lys Ala Asn Asn Arg Glu Arg Asn Arg Met His Asn Leu
           115                 120                 125
   Asn Ala Ala Leu Asp Ala Leu Arg Glu Val Leu Pro Thr Phe Pro Glu
       130                 135                 140
   Asp Ala Lys Leu Thr Lys Ile Glu Thr Leu Arg Phe Ala His Asn Tyr
   145                 150                 155                 160
   Ile Trp Ala Leu Thr Glu Thr Leu Arg Leu Ala Asp His Cys Gly Gly
                   165                 170                 175
   Gly Gly Gly Gly Leu Pro Gly Ala Leu Phe Ser Glu Ala Val Leu Leu
               180                 185                 190
   Ser Pro Gly Gly Ala Ser Ala Ala Leu Ser Ser Ser Gly Asp Ser Pro
           195                 200                 205
   Ser Pro Ala Ser Thr Trp Ser Cys Thr Asn Ser Pro Ala Pro Ser Ser
       210                 215                 220
   Ser Val Ser Ser Asn Ser Thr Ser Pro Tyr Ser Cys Thr Leu Ser Pro
   225                 230                 235                 240
   Ala Ser Pro Ala Gly Ser Asp Met Asp Tyr Trp Gln Pro Pro Pro Pro
                   245                 250                 255
   Asp Lys His Arg Tyr Ala Pro His Leu Pro Ile Ala Arg Asp Cys Ile
               260                 265                 270
<210> 2
   <211> 2370
   <212> DNA
   <213> Homo sapiens
<220>
   <221> source
   <222> 1..2370
   <223> /mol_type="DNA" /organism="Homo sapiens"
<400> 2
   cgcagccact gaaccacaag cagcttcgcg ttaactggag tgcctgggag tcgcgtgcca      60
   ggagccgcac ggccagggac tgactgacag acagacacgc accaccacca caacacacga     120
   gacccgggcg ggccgccgcc gccgccgccg gggctcttgg caaactcgcc ggtcgcagag     180
   gtcccccgcg gagctgcgcc acagtagcgc cgggcttgca gctttcacgc cgggcgaagg     240
   acccggcgct gcgctcgcag ctgcgcggag attcccggca caggccaaag tcacagcaac     300
   gctgaggcac agttagagcc aactaagatg ttcgtcaaat ccgagacctt ggagttgaag     360
   gaggaagagg acgtgttagt gctgctcgga tcggcctccc ccgccttggc ggccctgacc     420
   ccgctgtcat ccagcgccga cgaagaagag gaggaggagc cgggcgcgtc aggcggggcg     480
   cgtcggcagc gcggggctga ggccgggcag gggcgcgggg gcggcgtggc tgcgggtgcg     540
   gagggctgcc ggcccgcacg gctgctgggt ctggtacacg attgcaaacg gcgcccttcc     600
   cgggcgcggg ccgtctcccg aggcgccaag acggccgaga cggtgcagcg catcaagaag     660
   acccgtagac tgaaggccaa caaccgcgag cgaaaccgca tgcacaacct caacgcggca     720
   ctggacgcgc tgcgcgaggt gctccccacg ttccccgagg acgccaagct caccaagatc     780
   gagaccctgc gcttcgccca caactacatc tgggcactca ccgagaccct gcgcctggcg     840
   gatcactgcg gggcggcggc cgggggcctg ccgggggcgc tcttctccga ggcagtgttg     900
   ctgagcccgg gaggcgccag cgccgccctg agcagcagcg gagacagccc ctcgcccgcc     960
   tccacgtgga gttgcaccaa cagccccgcg ccgtcctcct ccgtgtcctc caattccacc    1020
   tccccctaca gctgcacttt atcgcccgcc agcccggccg ggtcagacat ggactattgg    1080
   cagcccccac ctcccgacaa gcaccgctat gcacctcacc tccccatagc cagggattgt    1140
   atctagagct gccatttctg ctacccacgc caggccttag tgggttccct ttcctgtccc    1200
   cagtcgagcc ctcctccctt cccctgcccc tcctttccac gccctggaaa ccatctcact    1260
   tcacagggca ggtgtagcct ttctgattcc tcggttgttt cttgcatttc ttggctttgg    1320
   gtatccttca ttcagacggg ctctgattta ctgaaggtgt gatggagctt attgtcaaag    1380
   ccaagggtgg cgttttgggg gcgcttcttg agacgaaaaa gaccctggga agagatgatg    1440
   gtggcatatc taaagagttt gcagagcgga ctgacgctcc tcccctttct ctttaacgcc    1500
   gaaggacttg gtgcagttcg tgtgaatctc acagggggaa tgcaactggt tcctgtgatc 1560
   tcttcacctt tgcttctaca tagagatgtt aatgtcgagt agaaagaaat gtatcttagc 1620
   atctgaatga ttttgctggt aataatatta tccacagatt tgcaatggct ggcatctgct 1680
   ttattcccat tgctgtctgc aggctgtggg aatttcacct gtcaaaccaa acttccctct 1740
   ctgatgtgca ctttgttctg tttcccagat tcgtcacaat gcctattgtc ctgtccttct 1800
   ctttcctttt tcttccccat tttgccatct gtctcttatg atttataagg ggaaaaaaac 1860
   ttgttttgtt agaggggcag gttagaagtc attgtataat ttgtaggctt tgtaatgatt 1920
   gaatgcaagc gtggaaattt aggctgaact ctctatcaaa aggaaaaatg tggaggaaaa 1980
   gggaaaaatc aggagggagg attgcctcat gtattattta tttcgacctt ttaggggaga 2040
   aggaactccc ccattctttc aagagattaa aaataaatca acagtctgaa aacctaagca 2100
   gacacggagc attatccgga tcagccacac acgtgttccc ttctatttat tataaagaaa 2160
   tttttcatgg gaaaatatgt attttttgta tattctacag agtttattct agtatgtatt 2220
   tacatcttga agaacaagaa agttgttctt gtgattaaac tataaataaa ctatctaatt 2280
   ttcataaaaa aaaaaaaaaa aaaaaaaaaa aaaaaaaaaa aaaaaaaaaa aaaaaaaaaa 2340
   aaaaaaaaaa aaaaaaaaaa aaaaaaaaaa 2370
<210> 3
   <211> 236
   <212> PRT
   <213> Homo sapiens
<220>
   <221> SOURCE
   <222> 1..236
   <223> /mol_type="protein" /organism="Homo sapiens"
<400> 3
   Met Glu Ser Ser Ala Lys Met Glu Ser Gly Gly Ala Gly Gln Gln Pro
   1               5                   10                  15
   Gln Pro Gln Pro Gln Gln Pro Phe Leu Pro Pro Ala Ala Cys Phe Phe
               20                  25                  30
   Ala Thr Ala Ala Ala Ala Ala Ala Ala Ala Ala Ala Ala Ala Ala Gln
           35                  40                  45
   Ser Ala Gln Gln Gln Gln Gln Gln Gln Gln Gln Gln Gln Gln Ala Pro
       50                  55                  60
   Gln Leu Arg Pro Ala Ala Asp Gly Gln Pro Ser Gly Gly Gly His Lys
   65                  70                  75                  80
   Ser Ala Pro Lys Gln Val Lys Arg Gln Arg Ser Ser Ser Pro Glu Leu
                   85                  90                  95
   Met Arg Cys Lys Arg Arg Leu Asn Phe Ser Gly Phe Gly Tyr Ser Leu
               100                 105                 110
   Pro Gln Gln Gln Pro Ala Ala Val Ala Arg Arg Asn Glu Arg Glu Arg
           115                 120                 125
   Asn Arg Val Lys Leu Val Asn Leu Gly Phe Ala Thr Leu Arg Glu His
       130                 135                 140
   Val Pro Asn Gly Ala Ala Asn Lys Lys Met Ser Lys Val Glu Thr Leu
   145                 150                 155                 160
   Arg Ser Ala Val Glu Tyr Ile Arg Ala Leu Gln Gln Leu Leu Asp Glu
                   165                 170                 175
   His Asp Ala Val Ser Ala Ala Phe Gln Ala Gly Val Leu Ser Pro Thr
               180                 185                 190
   Ile Ser Pro Asn Tyr Ser Asn Asp Leu Asn Ser Met Ala Gly Ser Pro
           195                 200                 205
   Val Ser Ser Tyr Ser Ser Asp Glu Gly Ser Tyr Asp Pro Leu Ser Pro
       210                 215                 220
   Glu Glu Gln Glu Leu Leu Asp Phe Thr Asn Trp Phe
   225                 230                 235
<210> 4
   <211> 2490
   <212> DNA
   <213> Homo sapiens
<220>
   <221> source
   <222> 1..2490
   <223> /mol_type="DNA" /organism="Homo sapiens"
<400> 4
   agcactctct cacttctggc cagggaacgt ggaaggcgca ccgacaggga tccggccagg      60
   gagggcgagt gaaagaagga aatcagaaag gaagggagtt aacaaaataa taaaaacagc     120
   ctgagccacg gctggagaga ccgagacccg gcgcaagaga gcgcagcctt agtaggagag     180
   gaacgcgaga cgcggcagag cgcgttcagc actgactttt gctgctgctt ctgctttttt     240
   ttttcttaga aacaagaagg cgccagcggc agcctcacac gcgagcgcca cgcgaggctc     300
   ccgaagccaa cccgcgaagg gaggagggga gggaggagga ggcggcgtgc agggaggaga     360
   aaaagcattt tcactttttt tgctcccact ctaagaagtc tcccggggat tttgtatata     420
   ttttttaact tccgtcaggg ctcccgcttc atatttcctt ttctttccct ctctgttcct      480
   gcacccaagt tctctctgtg tccccctcgc gggccccgca cctcgcgtcc cggatcgctc     540
   tgattccgcg actccttggc cgccgctgcg catggaaagc tctgccaaga tggagagcgg     600
   cggcgccggc cagcagcccc agccgcagcc ccagcagccc ttcctgccgc ccgcagcctg     660
   tttctttgcc acggccgcag ccgcggcggc cgcagccgcc gcagcggcag cgcagagcgc     720
   gcagcagcag cagcagcagc agcagcagca gcagcaggcg ccgcagctga gaccggcggc     780
   cgacggccag ccctcagggg gcggtcacaa gtcagcgccc aagcaagtca agcgacagcg     840
   ctcgtcttcg cccgaactga tgcgctgcaa acgccggctc aacttcagcg gctttggcta     900
   cagcctgccg cagcagcagc cggccgccgt ggcgcgccgc aacgagcgcg agcgcaaccg     960
   cgtcaagttg gtcaacctgg gctttgccac ccttcgggag cacgtcccca acggcgcggc    1020
   caacaagaag atgagtaagg tggagacact gcgctcggcg gtcgagtaca tccgcgcgct    1080
   gcagcagctg ctggacgagc atgacgcggt gagcgccgcc ttccaggcag gcgtcctgtc    1140
   gcccaccatc tcccccaact actccaacga cttgaactcc atggccggct cgccggtctc    1200
   atcctactcg tcggacgagg gctcttacga cccgctcagc cccgaggagc aggagcttct    1260
   cgacttcacc aactggttct gaggggctcg gcctggtcag gccctggtgc gaatggactt    1320
   tggaagcagg gtgatcgcac aacctgcatc tttagtgctt tcttgtcagt ggcgttggga    1380
   gggggagaaa aggaaaagaa aaaaaaaaga agaagaagaa gaaaagagaa gaagaaaaaa    1440
   acgaaaacag tcaaccaacc ccatcgccaa ctaagcgagg catgcctgag agacatggct    1500
   ttcagaaaac gggaagcgct cagaacagta tctttgcact ccaatcattc acggagatat    1560
   gaagagcaac tgggacctga gtcaatgcgc aaaatgcagc ttgtgtgcaa aagcagtggg    1620
   ctcctggcag aagggagcag cacacgcgtt atagtaactc ccatcacctc taacacgcac    1680
   agctgaaagt tcttgctcgg gtcccttcac ctcctcgccc tttcttaaag tgcagttctt    1740
   agccctctag aaacgagttg gtgtctttcg tctcagtagc ccccacccca ataagctgta    1800
   gacattggtt tacagtgaaa ctatgctatt ctcagccctt tgaaactctg cttctcctcc    1860
   agggcccgat tcccaaaccc catggcttcc ctcacactgt cttttctacc attttcatta    1920
   tagaatgctt ccaatctttt gtgaattttt tattataaaa aatctatttg tatctatcct    1980
   aaccagttcg gggatatatt aagatatttt tgtacataag agagaaagag agagaaaaat    2040
   ttatagaagt tttgtacaaa tggtttaaaa tgtgtatatc ttgatacttt aacatgtaat    2100
   gctattacct ctgcatattt tagatgtgta gttcacctta caactgcaat tttccctatg    2160
   tggttttgta aagaactctc ctcataggtg agatcaagag gccaccagtt gtacttcagc    2220
   accaatgtgt cttactttat agaaatgttg ttaatgtatt aatgatgtta ttaaatactg    2280
   ttcaagaaga acaaagttta tgcagctact gtccaaactc aaagtggcag ccagttggtt    2340
   ttgataggtt gccttttgga gatttctatt actgcctttt tttttcttac tgttttatta    2400
   caaacttaca aaaatatgta taaccctgtt ttatacaaac tagtttcgta ataaaacttt    2460
   ttcctttttt taaaatgaaa ataaaaaaaa                                     2490
<210> 5
   <211> 60
   <212> PRT
   <213> Artificial Sequence
<220>
   <221> SOURCE
   <222> 1..60
   <223> /mol_type="protein" /note="derived from NGN2 protein" /organism="Artificial Sequence"
<400> 5
   Lys Lys Thr Arg Arg Leu Lys Ala Asn Asn Arg Glu Arg Asn Arg Met
   1               5                   10                  15
   His Asn Leu Asn Ala Ala Leu Asp Ala Leu Arg Glu Val Leu Pro Thr
               20                  25                  30
   Phe Pro Glu Asp Ala Lys Leu Thr Lys Ile Glu Thr Leu Arg Phe Ala
           35                  40                  45
   His Asn Tyr Ile Trp Ala Leu Thr Glu Thr Leu Arg
       50                  55                  60
<210> 6
   <211> 41
   <212> PRT
   <213> Artificial Sequence
<220>
   <221> SOURCE
   <222> 1..41
   <223> /mol_type="protein" /note="derived from ASCL1 protein" /organism="Artificial Sequence"
<400> 6
   Asn Leu Gly Phe Ala Thr Leu Arg Glu His Val Pro Asn Gly Ala Ala
   1               5                   10                  15
   Asn Lys Lys Met Ser Lys Val Glu Thr Leu Arg Ser Ala Val Glu Tyr
               20                  25                  30
   Ile Arg Ala Leu Gln Gln Leu Leu Asp
           35                  40
<210> 7
   <211> 180
   <212> DNA
   <213> Artificial Sequence
<220>
   <221> source
   <222> 1..180
   <223> /mol_type="DNA" /note="derived from NGN2" /organism="Artificial Sequence"
<400> 7
   aagaagaccc gtagactgaa ggccaacaac cgcgagcgaa accgcatgca caacctcaac      60
   gcggcactgg acgcgctgcg cgaggtgctc cccacgttcc ccgaggacgc caagctcacc     120
   aagatcgaga ccctgcgctt cgcccacaac tacatctggg cactcaccga gaccctgcgc     180
<210> 8
   <211> 123
   <212> DNA
   <213> Artificial Sequence
<220>
   <221> source
   <222> 1..123
   <223> /mol_type="DNA" /note="derived from ASCL1" /organism="Artificial Sequence"
<400> 8
   aacctgggct ttgccaccct tcgggagcac gtccccaacg gcgcggccaa caagaagatg      60
   agtaaggtgg agacactgcg ctcggcggtc gagtacatcc gcgcgctgca gcagctgctg     120
   gac                                                                   123
<210> 9
   <211> 334
   <212> PRT
   <213> Homo sapiens
<220>
   <221> SOURCE
   <222> 1..334
   <223> /mol_type="protein" /organism="Homo sapiens"
<400> 9
   Met Thr Lys Ser Tyr Ser Glu Ser Gly Leu Met Gly Glu Pro Gln Pro
   1               5                   10                  15
   Gln Gly Pro Pro Ser Trp Thr Asp Glu Cys Leu Ser Ser Gln Asp Glu
               20                  25                  30
   Glu His Glu Ala Asp Lys Lys Glu Asp Asp Leu Glu Ala Met Asn Ala
           35                  40                  45
   Glu Glu Asp Ser Leu Arg Asn Gly Gly Glu Glu Glu Asp Glu Asp Glu
       50                  55                  60
   Asp Leu Glu Glu Glu Glu Glu Glu Glu Glu Glu Asp Asp Asp Gln Lys
   65                  70                  75                  80
   Pro Lys Arg Arg Gly Pro Lys Lys Lys Lys Met Thr Lys Ala Arg Leu
                   85                  90                  95
   Glu Arg Phe Lys Leu Arg Arg Met Lys Ala Asn Ala Arg Glu Arg Asn
               100                 105                 110
   Arg Met His Gly Leu Asn Ala Ala Leu Asp Asn Leu Arg Lys Val Val
           115                 120                 125
   Pro Cys Tyr Ser Lys Thr Gln Lys Leu Ser Lys Ile Glu Thr Leu Arg
       130                 135                 140
   Leu Ala Lys Asn Tyr Ile Trp Ala Leu Ser Glu Ile Leu Arg Ser Gly
   145                 150                 155                 160
   Lys Ser Pro Asp Leu Val Ser Phe Val Gln Thr Leu Cys Lys Gly Leu
                   165                 170                 175
   Ser Gln Pro Thr Thr Asn Leu Val Ala Gly Cys Leu Gln Leu Asn Pro
               180                 185                 190
   Arg Thr Phe Leu Pro Glu Gln Asn Gln Asp Met Pro Pro His Leu Pro
           195                 200                 205
   Thr Ala Ser Ala Ser Phe Pro Val His Pro Tyr Ser Tyr Gln Ser Pro
       210                 215                 220
   Gly Leu Pro Ser Pro Pro Tyr Gly Thr Met Asp Ser Ser His Val Phe
   225                 230                 235                 240
   His Val Lys Pro Pro Pro His Ala Tyr Ser Ala Ala Leu Glu Pro Phe
                   245                 250                 255
   Phe Glu Ser Pro Leu Thr Asp Cys Thr Ser Pro Ser Phe Asp Gly Pro
               260                 265                 270
   Leu Ser Pro Pro Leu Ser Ile Asn Gly Asn Phe Ser Phe Lys His Glu
           275                 280                 285
   Pro Ser Ala Glu Phe Glu Lys Asn Tyr Ala Phe Thr Met His Tyr Pro
       290                 295                 300
   Ala Ala Thr Leu Ala Gly Ala Gln Ser His Gly Ser Ile Phe Ser Gly
   305                 310                 315                 320
   Thr Ala Ala Pro Arg Cys Glu Ile Pro Ile Asp Asn Ile Met
                   325                 330
<210> 10
   <211> 1071
   <212> DNA
   <213> Homo sapiens
<220>
   <221> source
   <222> 1..1071
   <223> /mol_type="DNA" /organism="Homo sapiens"
<400> 10
   atgaccaaat cgtacagcga gagtgggctg atgggcgagc ctcagcccca aggtcctcca      60
   agctggacag acgagtgtct cagttctcag gacgaggagc acgaggcaga caagaaggag     120
   gacgacctcg aagccatgaa cgcagaggag gactcactga ggaacggggg agaggaggag     180
   gacgaagatg aggacctgga agaggaggaa gaagaggaag aggaggatga cgatcaaaag     240
   cccaagagac gcggccccaa aaagaagaag atgactaagg ctcgcctgga gcgttttaaa     300
   ttgagacgca tgaaggctaa cgcccgggag cggaaccgca tgcacggact gaacgcggcg     360
   ctagacaacc tgcgcaaggt ggtgccttgc tattctaaga cgcagaagct gtccaaaatc     420
   gagactctgc gcttggccaa gaactacatc tgggctctgt cggagatcct gcgctcaggc     480
   aaaagcccag acctggtctc cttcgttcag acgctttgca agggcttatc ccaacccacc     540
   accaacctgg ttgcgggctg cctgcaactc aatcctcgga cttttctgcc tgagcagaac     600
   caggacatgc ccccccacct gccgacggcc agcgcttcct tccctgtaca cccctactcc     660
   taccagtcgc ctgggctgcc cagtccgcct tacggtacca tggacagctc ccatgtcttc     720
   cacgttaagc ctccgccgca cgcctacagc gcagcgctgg agcccttctt tgaaagccct     780
   ctgactgatt gcaccagccc ttcctttgat ggacccctca gcccgccgct cagcatcaat     840
   ggcaacttct ctttcaaaca cgaaccgtcc gccgagtttg agaaaaatta tgcctttacc     900
   atgcactatc ctgcagcgac actggcaggg gcccaaagcc acggatcaat cttctcaggc     960
   accgctgccc ctcgctgcga gatccccata gacaatatta tgtccttcga tagccattca    1020
   catcatgagc gagtcatgag tgcccagctc aatgccatat ttcatgatta g             1071
<210> 11
   <211> 60
   <212> PRT
   <213> Artificial Sequence
<220>
   <221> SOURCE
   <222> 1..60 ,
   <223> /mol_type="protein" /note="derived from NEUROD1 protein" /organism="Artificial Sequence"
<400> 11
   Lys Leu Arg Arg Met Lys Ala Asn Ala Arg Glu Arg Asn Arg Met His
   1               5                   10                  15
   Gly Leu Asn Ala Ala Leu Asp Asn Leu Arg Lys Val Val Pro Cys Tyr
               20                  25                  30
   Ser Lys Thr Gln Lys Leu Ser Lys Ile Glu Thr Leu Arg Leu Ala Lys
           35                  40                  45
   Asn Tyr Ile Trp Ala Leu Ser Glu Ile Leu Arg Ser
       50                  55                  60
<210> 12
   <211> 180
   <212> DNA
   <213> Artificial Sequence
<220>
   <221> source
   <222> 1..180
   <223> /mol_type="DNA" /note="derived from NEUROD1" /organism="Artificial Sequence"
<400> 12
   aaattgagac gcatgaaggc taacgcccgg gagcggaacc gcatgcacgg actgaacgcg      60
   gcgctagaca acctgcgcaa ggtggtgcct tgctattcta agacgcagaa gctgtccaaa     120
   atcgagactc tgcgcttggc caagaactac atctgggctc tgtcggagat cctgcgctca     180

## Description

The present invention relates to bHLH proteins and their use as drugs.

Cancer stem cells (CSCs) are cancer cells (found within solid tumors or haematological cancers) that possess characteristics associated with normal stem cells, specifically the ability to give rise to all cell types found in determined cancer sample.

CSCs may generate tumors through the stem cell processes of self-renewal and differentiation into multiple cell types.

CSCs have been identified initially in leukaemia sample wherein an isolated subpopulation of leukaemic cells that express a specific surface marker CD34, but lacks the CD38 marker are capable of initiating tumors in NOD/SCID mice that is histologically similar to the donor.

The existence of leukaemic stem cells prompted further research into other types of cancer. CSCs have recently been identified in several solid tumors, including cancers of the breast, brain, colon, ovary, pancreas and prostate.

The efficacy of cancer treatments is measured by the reduction of the tumor mass. However, since CSCs form a small proportion of the tumor, they may not necessarily be targeted by the treatment. CSCs cells are proposed to persist in tumors as a distinct population and cause relapse and metastasis by giving rise to new tumors.

Therefore, there is a need to specifically eradicate cancer stem cells in order to limit relapse of tumors after remission, following antitumor therapies.

Gliomas are primitive tumours of the CNS which are derived from tumorigenesis of cells of the glial lineage (astrocytes and oligodendrocytes) (Louis, 2006, Annu Rev Pathol 1, 97-117; Behin, 2003, Lancet 361, 323-331). They are the most frequent brain tumours with an incidence of 1/20 000/inhabitants/year (3000 new cases in France, 15 000 news cases in US per year) (Bondy, 2008, Cancer. 2008 Oct 1;113(7 Suppl):1953-68; Bauchet 2007, J Neurooncol. 2007 Sep;84(2):189-99). These tumors are aggressive, highly invasive and neurologically destructive.

Glioma are divided in two main categories:
- High grade gliomas (grade III-IV according to WHO classification, Louis, 2007, Acta Neuropathol. 2007 Aug;114(2):97-109), which are mostly represented by multiform glioblastomas (GBM). These tumours contain highly proliferating cells and are associated with a very poor prognosis.
- Low grade gliomas (WHO grade II glioma, G2G), which growth slowly but which ineluctably evolves to anaplasia within 5-10 years.

One important feature of gliomas is their diffuse aspect due to migration and infiltration of the parenchyma from which deterioration will occur. There is currently no curative treatment for these tumors and despite maximum treatment efforts, median survival of patients diagnosed with GBM ranges from 9 to 12 months, a statistic that has changed very little in decades.

GBM are the most common glioma in humans (Kleihues 2000, Cancer. 2000 Jun 15; Maher, 2001, Genes Dev. 2001 Jun 1;15(11):1311-33) and can evolve from low grade glioma (secondary GBM) or develop de novo (primary GBM). Like all cancers, GBM share a relatively restricted set of characteristics crucial to their phenotype: proliferation in the absence of external growth stimuli, avoidance of apoptosis and no limits to replication, escape from both external growth-suppressive forces and the immune response, formation of new blood vessels and the ability to invade normal tissues (Hanahan and Weinberg 2000, Cell. 100(1):57-70). Furthermore, despite their striking heterogeneity, common alterations in specific cellular signal transduction pathways occur within most GBMs (Louis, 2006, Annu Rev Pathol 1, 97-117).

GBM may be derived from transformation of differentiated cells or alternatively of adult stem/progenitor cells (Dai 2003, Cancer J 9, 72-81; Holland, 2001, Curr Opin Neurol 14, 683-688).

Indeed, GBMs contain 1-20 % of cancer stem cells which grow on non adherent substrates to generate clonal expansion called neurospheres. The latter are multipotential and generate astrocytes and neuronal-like cells upon differentiation on adhesive substrate. These cancer stem cells appear to be more tumorigenic than the rest of tumoral cells when grafted in immunocompromised animals. In addition, these cells seem to be more chemo- and radio-resistant than the other tumoral cells.

As a consequence, new glioma drugs or treatments have to be found to specifically eradicate these cells.

Tumoral stem cells, like the non tumoral stem cells, reside in special vascular niches (Gilbertson, 2007, Nat Rev Cancer 7, 733-736) which provide high level of canonical stem cell signallings such as Wnt, Notch or SHH (Ischenko, 2008, Curr Med Chem. 2008;15(30):3171-84). These pathways maintain the cells in an undifferentiated state and contribute to their self-renewal.

In addition, it is now well documented that GBM cancer stem cells rely on a special set of genes (for instance Sox2, Olig2, Bmi1...) to maintain a high level of self-renewal. These genes could be considered as potential targets to specifically eliminate these cells.

The number of new molecules specifically developed to cure gliomas is very low. Treatments of the proliferative tumoral cells transiently reduce tumor progression. However, a relapse occurs, due to the persistence of tumoral stem cells.

One possible approach is to differentiate these CSCs into post-mitotic cells so as to turn off the proliferation program, and therefore limiting tumor growth. This approach has very satisfying results in Acute Promyelocytic leukemia (APL) treated with retinoic acid and/or arsenic.

This strategy requires a broad knowledge of the mechanisms and/or genes which are responsible for the differentiation of normal and tumoral stem cells. This has been partially elucidated for stem cells of the nervous system. Indeed, during development, these stem cells differentiate into neuronal cells as a consequence of the stabilisation and the increase of neurogenic transcription factors, notably those belonging to the bHLH family and in particular NGN1, NGN2, NGN3 and ASCL1 (Bertrand, 2002, Nat Rev Neurosci. Jul;3(7):517-30). This is well illustrated by gain and loss of function studies. Indeed, overexpression of *NGN2* in the neural tube of chick embryos results in premature cell cycle exit and premature neuronal differentiation of neuroepithelial stem cells, (Mizugushi, 2001 Neuron 31:757-771; Sugimori 2007 Development, 134(8):1617-29). Conversely double mutant animals for NGN1 and 2 present a marked reduction in neurogenesis (Scardigli, 2001, Neuron 31.203-217*).* Likewise, overexpression of ASCL1 in cerebellum promotes cell-cycle exit and differentiation (Alvarez-Rodríguez 2009 J Cell Sci. Mar 1;122, 595-9) while ASCL1 loss of function results in impaired neurogenesis of postnatal neural stem cells (EMBO J. 2004 Nov 10;23(22):4495-505.).

In this context, some of the above genes have been used for the treatment of different pathologies.

For instance, WO 2004/016779 discloses pharmaceutical compositions comprising NGN2 or ASCL1 for their use in cell therapy of neurological diseases.

US 2004/152168 proposes compositions comprising NGN2 or ASCL1 for treating insulin associated disorders.

WO 01/26643 discloses a method for inhibiting tumorigenic properties of melanoma cells by using bHLH proteins.

An alternative possibility to eliminate cancer stem cells is to inactivate one or several stem cell signallings with specific drugs targeting these pathways (Ischenko, 2008, Curr Med Chem. 2008;15(30):3171-84).

Last, one can also consider the possibility of targeting key stem cell genes to eradicate the source of the tumor.

The need of an efficient drug able to completely and efficiently eradicate cancer cells, and cancer stem cells, remains.

Therefore, one aim of the invention is to provide a new efficient drug for treating pathologies, including cancer.

Another aim of the invention is to provide an efficient therapy for treating tumors, without risk of relapse.

The present disclosure relates to a composition comprising
- at least one protein belonging to the bHLH family, chosen among NGN2, or ASCL1, and NEUROD1 and/or
- at least a nucleic acid molecule coding for said bHLH proteins, i.e. coding for NGN2, or ASCL1, and NEUROD1 proteins, as drug inducing cell death, preferably as apoptotic drug.

The present disclosure relates to a composition comprising
- at least one protein belonging to the bHLH family, chosen among NGN2, ASCL1, and NEUROD1 and/or
- at least a nucleic acid molecule coding for said bHLH proteins, i.e. coding for NGN2, ASCL1, and NEUROD1 proteins, for its use for inducing apoptosis of cancer cells, preferably for inducing apoptosis of cancer stem cells.

The present invention relates to a composition comprising:
- at least one protein belonging to the bHLH family, chosen among NGN2, ASCL1, and NEUROD1 and/or
- at least a nucleic acid molecule coding for said bHLH proteins, for its use for the treatment of central nervous system (CNS) tumors and neuroendocrine tumors.

In other words the disclosure_relates to a composition comprising
- either at least one protein belonging to the bHLH family, chosen among NGN2, ASCL1, and NEUROD1,
- or at least a nucleic acid molecule coding for said bHLH proteins, i.e. coding for NGN2, ASCL1, and NEUROD1 proteins proteins,
- or at least one protein belonging to the bHLH family, chosen among NGN2, ASCL1, and NEUROD1, and at least a nucleic acid molecule coding for said bHLH proteins, i.e. coding for NGN2, ASCL1, and NEUROD1 proteins,

for its use for inducing apoptosis of cancer cells, preferably for inducing apoptosis of cancer stem cells.

In other words the disclosure relates to a composition comprising
- either at least one protein belonging to the bHLH family, chosen among NGN2, ASCL1, and NEUROD1,
- or at least a nucleic acid molecule coding for said bHLH proteins, i.e. coding for NGN2, ASCL1, and NEUROD1 proteins proteins,
- or at least one protein belonging to the bHLH family, chosen among NGN2, ASCL1, and NEUROD1, and at least a nucleic acid molecule coding for said bHLH proteins, i.e. coding for NGN2, ASCL1, and NEUROD1 proteins,

for its use for the treatment of central nervous system (CNS) tumors and neuroendocrine tumors.

Terms "at least one" or "at least a" refer, in the invention, to one, or two, or three, or more.

The 63 possibilities, covered by the invention, are represented by the following table 1:

**Table 1**

| | NGN2 | | | ASCL1 | | | NeuroD1 | | |
|---|---|---|---|---|---|---|---|---|---|
| Composi-tion n° | P | D | P+D | P | D | P+D | P | D | P+D |
| 1 | + | | | | | | | | |
| 2 | | + | | | | | | | |
| 3 | | | + | | | | | | |
| 4 | | | | + | | | | | |
| 5 | | | | | + | | | | |
| 6 | | | | | | + | | | |
| 7 | | | | | | | + | | |
| 8 | | | | | | | | + | |
| 9 | | | | | | | | | + |
| 10 | + | | | + | | | | | |
| 11 | + | | | | + | | | | |
| 12 | + | | | | | + | | | |
| 13 | + | | | | | | + | | |
| 14 | + | | | | | | | + | |
| 15 | + | | | | | | | | + |
| 16 | | + | | + | | | | | |
| 17 | | + | | | + | | | | |
| 18 | | + | | | | + | | | |
| 19 | | + | | | | | + | | |
| 20 | | + | | | | | | + | |
| 21 | | + | | | | | | | + |
| 22 | | | + | + | | | | | |
| 23 | | | + | | + | | | | |
| 24 | | | + | | | + | | | |
| 25 | | | + | | | | + | | |
| 26 | | | + | | | | | + | |
| 27 | | | + | | | | | | + |
| 28 | | | | + | | | + | | |
| 29 | | | | + | | | | + | |
| 30 | | | | + | | | | | + |
| 31 | | | | | + | | + | | |
| 32 | | | | | + | | | + | |
| 33 | | | | | + | | | | + |
| 34 | | | | | | + | + | | |
| 35 | | | | | | + | | + | |
| 36 | | | | | | + | | | + |
| 37 | + | | | + | | | + | | |
| 38 | + | | | | + | | + | | |
| 39 | + | | | | | + | + | | |
| 40 | + | | | + | | | | + | |
| 41 | + | | | | + | | | + | |
| 42 | + | | | | | + | | + | |
| 43 | + | | | + | | | | | + |
| 44 | + | | | | + | | | | + |
| 45 | + | | | | | + | | | + |
| 46 | | + | | + | | | + | | |
| 47 | | + | | | + | | + | | |
| 48 | | + | | | | + | + | | |
| 49 | | + | | + | | | | + | |
| 50 | | + | | | + | | | + | |
| 51 | | + | | | | + | | + | |
| 52 | | + | | + | | | | | + |
| 53 | | + | | | + | | | | + |
| 54 | | + | | | | + | | | + |
| 55 | | | + | + | | | + | | |
| 56 | | | + | | + | | + | | |
| 57 | | | + | | | + | + | | |
| 58 | | | + | + | | | | + | |
| 59 | | | + | | + | | | + | |
| 60 | | | + | | | + | | + | |
| 61 | | | + | + | | | | | + |
| 62 | | | + | | + | | | | + |
| 63 | | | + | | | + | | | + |

Table 1 represents the 63 possibilities of the composition used according to the invention. P: Protein; D:DNA; P+D: Protein + DNA.

The invention is based on the unexpected observation made by the Inventors that the over-expression of NGN2, ASCL1 or NEUROD1 proteins induces cell death. Surprisingly, other bHLH proteins such as NGN1 and NGN3 are not able to induce cell death when their expression is enforced in cells.

Moreover, the above mentioned over-expression of NGN2, ASCL1 or NEUROD1 proteins also induces enforced differentiation of tumoral cells into functional differentiated cells.

For instance, a tumor derived from central nervous system can be enforced to differentiate toward neurone that is electro-physiologically active (See example section).

The expression "a nucleic acid molecule coding for bHLH proteins" refers to the NGN2, ASCL1 or NEUROD1 genes or the corresponding transcripts (RNA transcript of the NGN2, ASCL1, or NEUROD1 genes).

By convention, the name of proteins is capitalized (e.g.NGN2) and the corresponding gene coding for said protein is represented by slanting characters (e.g. NGN2).

The composition for use according to the invention kills cells, in particular tumoral cells, more preferably cancer stem cells.

There are many possibilities to kill cells: by inducing programmed cellular death (also called apoptosis), by inducing necrosis, or by inducing autophagy.

Autophagy, or autophagocytosis, is a catabolic process involving the degradation of a cellular own components through the lysosomal machinery. It is a tightly-regulated process that plays a normal part in cell growth, development, and homeostasis, helping to maintain a balance between the synthesis, degradation, and subsequent recycling of cellular products. It is a major mechanism by which a starving cell reallocates nutrients from unnecessary processes to more-essential processes.

Apoptosis is the process of programmed cell death that may occur in multicellular organisms. Biochemical events lead to characteristic cell changes (morphology) and death. These changes include blebbing, loss of cell membrane asymmetry and attachment, cell shrinkage, nuclear fragmentation, chromatin condensation, and chromosomal DNA fragmentation.

Necrosisis corresponds to the premature death of cells and living tissue. Necrosis is caused by factors external to the cell or tissue, such as infection, toxins, or trauma. This is in contrast to apoptosis, which is a naturally occurring cause of cellular death. While apoptosis often provides beneficial effects to the organism, necrosis is almost always detrimental and can be fatal.

The composition for use according to the invention is able to kill cells rapidly and efficiently, as illustrated in the Example section.

The composition as defined above is used as apoptotic drug.

"Apoptotic drug" according to the invention defines a drug having properties to induce programmed cell death of cells treated with said drug.

Apoptosis can be easily measured by general protocols known in the art. These protocols include, for instance:
- annexin V detection at the cell surface, by using flow cytometry
- caspase activation measurement, such as Caspase 3 activation
- DNA fragmentation measurement, for instance by TUNNEL method,
- Cytochrome C measurement.

It is also possible to measure apoptosis by flow cytometry (FACS) by measuring the DNA content, in particular by quantifying the population of cells having a DNA content lower than the DNA content of a diploid cell (sub G1 population).

The skilled person is able also to measure cell apoptosis by other well described methods disclosed in the art.

In one advantageous embodiment, the invention relates to a composition for use as defined above, wherein said NGN2 protein comprises or consists of
- the amino acid sequence SEQ ID NO:1, or
- any amino acid sequence having at least 85% of identity with the amino acid sequence SEQ ID NO : 1, preferably any amino acid sequence having at least 90% of identity with the amino acid sequence SEQ ID NO:1, or
- a fragment of said amino acid molecules provided that said fragments induce cell death, in particular apoptosis, preferably said fragments comprising or being constituted by the amino acid sequence SEQ ID NO: 5.

In one other advantageous embodiment, the invention relates to the composition for use as defined above, wherein said ASCL1 protein comprises or consists of
- the amino acid sequence SEQ ID NO:2, or
- any amino acid sequence having at least 85% of identity with the amino acid sequence SEQ ID NO :2, preferably any amino acid sequence having at least 90% of identity with the amino acid sequence SEQ ID NO:2,
- a fragment of said amino acid molecules provided that said fragments induce cell death, in particular apoptosis, preferably said fragments comprising or being constituted by the amino acid sequence SEQ ID NO: 6.

In one advantageous embodiment, the invention relates to a composition for use as defined above, wherein said NEUROD1 protein comprises or consists of
- the amino acid sequence SEQ ID NO:9, or
- any amino acid sequence having at least 85% of identity with the amino acid sequence SEQ ID NO :9, preferably any amino acid sequence having at least 90% of identity with the amino acid sequence SEQ ID NO:9, or
- a fragment of said amino acid molecules provided that said fragments induce cell death, in particular apoptosis, preferably said fragments comprising or being constituted by the amino acid sequence SEQ ID NO: 11.

In another advantageous embodiment, the invention relates to a composition for use as previously defined, wherein said nucleic acid molecule coding for NGN2 protein comprises or consists of
- the nucleic acid sequence SEQ ID NO : 3, or
- any nucleic acid molecule having at least 75%, preferably at least 85%, more preferably at least 95% of homology with the nucleic acid sequence SEQ ID NO : 3, or
- a fragment of nucleic acid molecules provided that said fragments induce cell death, in particular apoptosis, preferably said fragments comprising or being constituted by the amino acid sequence SEQ ID NO 7, coding for the fragment comprising or being constituted by the amino acid sequence SEQ ID NO : 5 as defined above.

In another advantageous embodiment, the invention relates to a composition for use as defined above, wherein said nucleic acid molecule coding for ASCL1 protein comprises or consists of
- the nucleic acid sequence SEQ ID NO : 4, or
- any nucleic acid molecule having at least 75%, preferably at least 85%, more preferably at least 95% of homology with the nucleic acid sequence SEQ ID NO : 4,
- a fragment of said nucleic acid molecules provided that said fragments induce cell death, in particular apoptosis, preferably said fragments comprising or being constituted by the nucleic acid sequence SEQ ID NO 8, coding for the fragment comprising the amino acid sequence SEQ ID NO : 6 as defined above.

In another advantageous embodiment, the invention relates to a composition for use as previously defined, wherein said nucleic acid molecule coding for NEUROD1 protein comprises or consists of
- the nucleic acid sequence SEQ ID NO : 10, or
- any nucleic acid molecule having at least 75%, preferably at least 85%, more preferably at least 95% of homology with the nucleic acid sequence SEQ ID NO : 10, or
- a fragment of nucleic acid molecules provided that said fragments induce cell death, in particular apoptosis, preferably said fragments comprising or being constituted by the amino acid sequence SEQ ID NO 10, coding for the fragment comprising or being constituted by the amino acid sequence SEQ ID NO : 12 as defined above.

Another embodiment according to the invention relates to a composition for use as defined above, wherein each of said nucleic acid molecule is comprised, or contained, in at least one vector, said vector comprising nucleic acid sequences allowing the expression of said nucleic acid molecule.

As mentioned above, the nucleic acid molecule coding for NGN2, ASCL1, or NEUROD1 proteins, or fragments thereof, as defined above, is contained in a vector containing specific sequences allowing :
- its replication in a lower eukaryote, such as bacteria,
- the initiation of transcription,
- termination of transcription, and
- antibiotics resistance, or drug susceptibility.

Moreover said vector contains any other sequences necessary for an expression in an host organism.

By "nucleic acid sequences allowing the expression of said nucleic acid molecule", the invention refers to nucleic acid sequences allowing the initiation of transcription, i.e. promoter, and nucleic acid sequences allowing the termination of transcription.

In order to allow the expression in mammal cells, such as human cells, the nucleic acid sequence according to the invention can be placed under the control of promoter sequences such as Cytomegalovirus (CMV) promoter, Rous Sarcoma Virus (RSV) promoter, Elongation Factor 1α (EF1-α) promoter or Ubiquitin gene (Ub) promoter. All these promoters allow the expression whatsoever the cell.

The nucleic acid molecule mentioned above can be also placed under the control of Long Terminal repeat (LTR) promoters derived from retroviruses or lentiviruses.

All these promoters are known in the art, and the skilled person can easily choose the best promoter for controlling the expression of the nucleic acid molecules coding for NGN2, ASCL1, and NEUROD1 proteins or fragments thereof.

Vectors to deliver and express the nucleic acid molecule into tumoral stem cells are for instances, HIV-1 derived lentiviral vectors, either carrying a wild type or a mutant (D64V) integrase, leading respectively to integrating (Trip-PGK) and non-integrating (Ni-Trip-PGK) vectors (Sarky, 2008, Curr Gene Ther. 8(6):430-7).

Non integrative lentivirus allows gene transfer to the nucleus and its expression without integration in the genome. The viral genomes remain as episomes which lack replication signals and are thus progressively diluted out. This allows a "hit and run" mode of gene expression and represents a significant safety benefits over their integrative counterparts.

In addition to lentiviruses, adenovirus vectors such as canine adenovirus vectors (Paul, 2008, Cancer Biol Ther. 2008 May;7(5):786-93), can be also used in the invention for targeting glioma cells.

In one other embodiment, the invention relates to a composition for use as mentioned above, comprising a vector comprising:
- a first nucleic acid molecule coding for said NGN2 protein, or fragments thereof, and
- a second nucleic acid molecule coding for said ASCL1 protein, or fragments thereof,
wherein both first and second nucleic acid molecule are placed under the control of nucleic acid sequences allowing the expression of said nucleic acid molecules.

In this embodiment, both nucleic acid molecules are contained in the same vector and their expression is governed by one promoter. For this purpose, the above first and above second nucleic acid molecules are linked by an Internal Ribosomal Entry Site (IRES) sequence that allow the transcription in a unique transcript of 2 sequences, but will provide the transcription of two separated and independent proteins.

In one other embodiment, the invention relates to a composition for use as mentioned above, comprising a vector comprising:
- a first nucleic acid molecule coding for said NGN2 protein, or fragments thereof, and
- a second nucleic acid molecule coding for said NEUROD1 protein, or fragments thereof,
wherein both first and second nucleic acid molecule are placed under the control of nucleic acid sequences allowing the expression of said nucleic acid molecules.

In this embodiment, both nucleic acid molecules are contained in the same vector and their expression is governed by one promoter. For this purpose, the above first and above second nucleic acid molecules are linked by an Internal Ribosomal Entry Site (IRES) sequence that allow the transcription in a unique transcript of 2 sequences, but will provide the translation of two separated and independent proteins.

In another embodiment, the invention relates to a composition for use as mentioned above, comprising a vector comprising:
- a first nucleic acid molecule coding for said ASCL1 protein, or fragments thereof, and
- a second nucleic acid molecule coding for said NEUROD1 protein, or fragments thereof,
wherein both first and second nucleic acid molecule are placed under the control of nucleic acid sequences allowing the expression of said nucleic acid molecules.

In this embodiment, both nucleic acid molecules are contained in the same vector and their expression is governed by one promoter. For this purpose, the above first and above second nucleic acid molecules are linked by an Internal Ribosomal Entry Site (IRES) sequence that allow the transcription in a unique transcript of 2 sequences, but will provide the translation of two separated and independent proteins.

In still another embodiment, the invention relates to a composition for use as mentioned above, wherein:
- a first nucleic acid molecule coding for said NGN2 protein, or fragments thereof,
- a second nucleic acid molecule coding for said ASCL1 protein, or fragments thereof, and
- a third nucleic acid molecule coding for said NEUROD1 protein, or fragments thereof,
are comprised in the same vector,
wherein first, second and third nucleic acid molecule are placed under the control of nucleic acid sequences allowing the expression of said nucleic acid molecules.

In this embodiment, the three nucleic acid molecules are contained in the same vector and their expression is governed by one promoter. For this purpose, the above first, second and third second nucleic acid molecules are linked by an Internal Ribosomal Entry Site (IRES) sequence that allow the transcription in a unique transcript of 3 sequences, but will provide the translation of three separated and independent proteins.

In one other embodiment, the invention relates to a composition for use as defined above, wherein
- at least one protein belonging to the bHLH family, chosen among NGN2, ASCL1 and NEUROD1 and/or
- at least a nucleic acid molecule coding for said bHLH proteins, are used in a simultaneous, separate or sequential manner.

In one other embodiment, the composition corresponds to one of the compositions disclosed above in table 1 for use as defined above, wherein the component of said composition are to be used in a simultaneous, separate or sequential manner.

Thus, in this embodiment, the invention relates to a composition for use as defined above, comprising the following components:
- NGN2 protein and ASCL1 protein, or
- nucleic acid molecule coding for NGN2 protein and ASCL1 protein, or
- NGN2 protein, nucleic acid molecule coding for NGN2 protein and ASCL1 protein, or
- NGN2 protein and nucleic acid molecule coding for ASCL1 protein, or
- nucleic acid molecule coding for NGN2 protein and nucleic acid molecule coding for ASCL1 protein, or
- NGN2 protein, nucleic acid molecule coding for NGN2 protein and nucleic acid molecule coding for ASCL1 protein, or
- NGN2 protein, ASCL1 protein and nucleic acid molecule coding for ASCL1 protein, or
- nucleic acid molecule coding for NGN2 protein, ASCL1 protein and nucleic acid molecule coding for ASCL1 protein, or
- NGN2 protein, nucleic acid molecule coding for NGN2 protein, ASCL1 protein and nucleic acid molecule coding for ASCL1 protein,
said components being used in a simultaneous, separate or sequential manner.

In one other embodiment, the invention relates to the composition corresponding to one of the compositions 10 to 63 disclosed above in table 1 for use as defined above, wherein the component of said composition are used in a simultaneous, separate or sequential manner.

From table 1, the skilled person knows what are the components of said composition. For instance ;
- composition 10 of table 1 comprises: NGN2 and ASCL1 protein,
- composition 24 of table 1 comprises: NGN2 protein and nucleic acid coding for NGN2 protein, and ASCL1 protein and nucleic acid coding for ASCL1 protein,
- composition 47 of table 1 comprises : nucleic acid coding for NGN2 protein, nucleic acid coding for ASCL1 protein, and NEUROD1 protein,
- composition 59 of table 1 comprises: NGN2 protein and nucleic acid coding for NGN2 protein, and nucleic acid coding for ASCL1 protein, and NEUROD1 protein and nucleic acid coding for NEUROD1 protein, and
- composition 63 of table 1 comprises: NGN2 protein and nucleic acid coding for NGN2 protein, and ASCL1 protein and nucleic acid coding for ASCL1 protein, and NEUROD1 protein and nucleic acid coding for NEUROD1 protein.

In one other embodiment, the invention relates to a composition for use as defined above, further comprising at least one antitumoral agent.

By "antitumoral agent", it is defined in the invention a compound or a drug commonly used for treating cancer in the frame of chemotherapy.

The majority of antitumoral agents can be divided into alkylating agents, antimetabolites, anthracyclines, plant alkaloids, topoisomerase inhibitors. These agents commonly interfere with cell division and DNA replication, and therefore limiting the multiplication of cancer cells.

Some advantageous antitumoral agents used according to the invention are cisplatin, vincristin, vinblastin, taxanes compounds or ectoposides.

In still another embodiment, the invention relates to a composition for use as defined above, wherein
- at least one protein belonging to the bHLH family, chosen among NGN2, ASCL1 and NEUROD1, and/or
- at least a nucleic acid molecule coding for said bHLH proteins, and
- said least one antitumoral agent
are used in a simultaneous, separate or sequential manner.

Advantageously, the invention relates to a composition for use as defined above, wherein said composition comprises:
either
   - at least two protein belonging to the bHLH family, chosen among NGN2, ASCL1, and NEUROD1,
      or
   - at least two nucleic acid molecule coding for said bHLH proteins,
      or
   - at least one protein belonging to the bHLH family, chosen among NGN2, ASCL1, and NEUROD1, and
   - at least a nucleic acid molecule coding for said bHLH proteins, which are used in a simultaneous, separate or sequential manner.

Terms "at least two protein belonging to the bHLH family, chosen among NGN2, ASCL1, and NEUROD1" means NGN2 and ASCL1 protein, NGN2 and NEUROD1 proteins, ASCL1 and NEUROD1 proteins and NGN2, ASCL1 and NEUROD1 proteins.

Terms "at least two nucleic acid molecule coding for said bHLH proteins" means nucleic acid molecules coding for NGN2 and ASCL1 protein, nucleic acid molecules coding for NGN2 and NEUROD1 proteins, nucleic acid molecules coding for ASCL1 and NEUROD1 proteins and nucleic acid molecules coding for NGN2, ASCL1 and NEUROD1 proteins.

Another embodiment according to the invention relates to a composition for use as previously defined, in association with a pharmaceutically acceptable carrier.

The appropriate pharmaceutically acceptable carrier is determined by the skilled person.

For instance, if the composition for use according to the invention contains proteins, said composition can be in a form of liposome, microsphere carriers, or the protein can be in a form of fusion protein with VIH TAT protein or Protein Transduction Domain (PTD) of viral proteins.

The above mentioned carriers are such that they allow the delivery to, and the entry into, the target cell of the protein contained in the composition used according to the invention.

In particular, pharmaceutically acceptable carrier allows crossing the blood brain barrier (BBB).

For instance, nucleic acid molecules are encapsulated in a 100 nm pegylated liposome and conjugated with receptor specific targeting monoclonal antibodies can cross the BBB and target tumoral cells (Pardridge, 2007, Pharm Res.24(9): 1733-44)

For instance, if the composition for use according to the invention contains nucleic acid molecules, carriers can be liposome, microsphere carriers or inactivated viral particles, that allow the penetration of the nucleic acid molecule into the target cell.

These examples are not limitative.

Dosage of the active substance depends on the administration route, and can be easily determined by a skilled person. The pharmaceutical composition for use according to the invention can be administered by intravenous route, sub-cutaneous route, systemic route, or can be administered locally by infiltration, or per os.

The pharmaceutical composition for use according to the invention can be administered at a dosage from about 0.001 g/kg/day to about 0.1 g/kg/day, according to the administration route.

In particular, the pharmaceutical compositions for use according to the invention may be administered at a dosage from about 0.05 to about 5g/day in adults, or from about 0.01 to about 1g/day for children.

In a particular embodiment, the pharmaceutical composition for use according to the invention contains at least a compound as previously defined in a form of the pharmaceutically acceptable salts known to a person skilled in the art, such as sodium salts, ammonium salts, calcium salts, magnesium salts, potassium salts, acetate salts, carbonate salts, citrate salts, chloride salts, sulphate salts, amino chlorhydate salts, borhydrate salts, phosphate salts, dihydrogenophosphate salts, succinate salts, citrate salts, tartrate salts, lactate salts, mandelate salts, methane sulfonate salts (mesylate) or p-toluene sulfonate salts (tosylate).

Also, the disclosure relates to the use of a composition, preferably an apoptotic composition, as defined above, for the preparation of a drugs intended for the treatment of central nervous system (CNS) tumors and neuroendocrine tumors.

The invention also relates to a composition, preferably an apoptotic composition, as defined above, comprising
- at least one protein belonging to the bHLH family, chosen among NGN2, ASCL1 and NEUROD1, or fragment thereof, and/or
- at least a nucleic acid molecule coding for said bHLH proteins, i.e. coding for NGN2, ASCL1 and NEUROD1 proteins, or fragment thereof,
for its use for the treatment of central nervous system (CNS) tumors and neuroendocrine tumors.

An advantageous embodiment of the invention relates to a composition for use as defined above, wherein
said NGN2 protein comprises or consists of
- the amino acid sequence SEQ ID NO:1, or
- any amino acid sequence having at least 85% of identity with the amino acid sequence SEQ ID NO :1, preferably any amino acid sequence having at least 90% of identity with the amino acid sequence SEQ ID NO:1, or
- a fragment of said amino acid molecules provided that said fragments induce cell death, in particular apoptosis, preferably said fragments comprising or being constituted by the amino acid sequence SEQ ID NO: 5,
   and/or
   said ASCL1 protein comprises or consists of
- the amino acid sequence SEQ ID NO:2, or
- any amino acid sequence having at least 85% of identity with the amino acid sequence SEQ ID NO :2, preferably any amino acid sequence having at least 90% of identity with the amino acid sequence SEQ ID NO:2,
- a fragment of said amino acid molecules provided that said fragments induce cell death, in particular apoptosis, preferably said fragments comprising or being constituted by the amino acid sequence SEQ ID NO: 6,
   and/or
   said NEUROD1 protein comprises or consists of
- the amino acid sequence SEQ ID NO:9, or
- any amino acid sequence having at least 85% of identity with the amino acid sequence SEQ ID NO :9, preferably any amino acid sequence having at least 90% of identity with the amino acid sequence SEQ ID NO:9, or
- a fragment of said amino acid molecules provided that said fragments induce cell death, in particular apoptosis, preferably said fragments comprising or being constituted by the amino acid sequence SEQ ID NO: 11,
   and/or
   said nucleic acid molecule coding for NGN2 protein comprise or consists of
- the nucleic acid sequence SEQ ID NO : 3, or
- any nucleic acid molecule having at least 75%, preferably at least 85%, more preferably at least 95% of homology with the nucleic acid sequence SEQ ID NO : 3,
- a fragment of nucleic acid molecules provided that said fragments induce cell death, in particular apoptosis, preferably said fragments comprising or being constituted by the amino acid sequence SEQ ID NO 7, coding for the fragment comprising or being constituted by the amino acid sequence SEQ ID NO : 5 as defined above,
   and/or
   said nucleic acid molecule coding for ASCL1 protein comprise or consists of
- the nucleic acid sequence SEQ ID NO : 4, or
- any nucleic acid molecule having at least 75%, preferably at least 85%, more preferably at least 95% of homology with the nucleic acid sequence SEQ ID NO : 2,
- a fragment of said nucleic acid molecules provided that said fragments induce cell death, in particular apoptosis, preferably said fragments comprising or being constituted by the nucleic acid sequence SEQ ID NO 8, coding for the fragment comprising the amino acid sequence SEQ ID NO : 6 as defined above,
   and/or
   said nucleic acid molecule coding for NEUROD1 protein comprise or consists of
- the nucleic acid sequence SEQ ID NO : 10, or
- any nucleic acid molecule having at least 75%, preferably at least 85%, more preferably at least 95% of homology with the nucleic acid sequence SEQ ID NO : 10,
- a fragment of nucleic acid molecules provided that said fragments induce cell death, in particular apoptosis, preferably said fragments comprising or being constituted by the amino acid sequence SEQ ID NO 10, coding for the fragment comprising or being constituted by the amino acid sequence SEQ ID NO : 12 as defined above.

Another advantageous embodiment of the invention relates to the composition for use as defined above, wherein said composition comprises

either each of said at least a nucleic acid molecule is comprised, or contained, in at least one vector, said vector comprising nucleic acid sequences allowing the expression of said nucleic acid molecule, or at least a vector comprising:
- a first nucleic acid molecule coding for said NGN2 protein, or fragments thereof, and
   a second nucleic acid molecule coding for said ASCL1 protein, or fragments thereof,
   wherein both first and second nucleic acid molecule are placed under the control of nucleic acid sequences allowing the expression of said nucleic acid molecules, or
   at least a vector comprising:
- a first nucleic acid molecule coding for said NGN2 protein, or fragments thereof, and
- a second nucleic acid molecule coding for said NEUROD1 protein, or fragments thereof,
   wherein both first and second nucleic acid molecule are placed under the control of nucleic acid sequences allowing the expression of said nucleic acid molecules, or
   at least a vector comprising:
- a first nucleic acid molecule coding for said NEUROD1 protein, or fragments thereof, and
- a second nucleic acid molecule coding for said ASCL1 protein, or fragments thereof,
   wherein both first and second nucleic acid molecule are placed under the control of nucleic acid sequences allowing the expression of said nucleic acid molecules, or
   at least a vector comprising:
- a first nucleic acid molecule coding for said NGN2 protein, or fragments thereof,
- a second nucleic acid molecule coding for said ASCL1 protein, or fragments thereof, and
- a third nucleic acid molecule coding for said NEUROD1 protein, or fragments thereof,
wherein said first, second and third nucleic acid molecules are placed under the control of nucleic acid sequences allowing the expression of said nucleic acid molecules.

The disclosure relates to the use of the above composition for the preparation of a drug in the frame of the treatment of central nervous system (CNS) tumors and neuroendocrine tumors, said composition comprising:
- at least one protein belonging to the bHLH family, chosen among NGN2, ASCL1, and NEUROD1 and/or
- at least a nucleic acid molecule coding for said bHLH proteins, as defined above, advantageously in the advantageous embodiments.

The invention relates to a composition as defined above for its use for the treatment, or for the preparation of a drug in the frame of the treatment of central nervous system (CNS) tumors and neuroendocrine tumors.

Advantageous embodiment relates to
- a composition for use as defined above, or
- the use of said composition for the preparation of a drug as defined above,
wherein said CNS tumors are chosen among the group consisting of grade II-IV glioma according to the 2007 WHO classification of tumours of the central nervous system.

The WHO classification is known in the art (Acta Neuropathol. 2007 Aug;114(2):97-109). Grade II-IV glioma refer to grade II glioma, grade III glioma and grade IV glioma.

Advantageous embodiment discloses herein relates to
- a composition used as defined above, or
- the use of said composition for the preparation of a drug as defined above,
wherein said neuroendocrines tumors are chosen among the group consisting of primary or metastatic gastro-entero-pancreatic neuroendocrine tumors (Massironi , 2008, World J Gastroenterol. 14(35):5377-84) as defined by the WHO classification (Solcia, 2000, Berlin: Springer;. pp. 56-70).

The disclosure relates to a method for treating central nervous system (CNS) tumors and neuroendocrine tumors, in particular grade II-IV glioma according to the 2007 WHO classification of tumours of the central nervous system or primary or metastatic gastro-entero-pancreatic neuroendocrine tumors, comprising the administration to a patients in a need thereof of a pharmaceutically effective amount of a composition comprising
- at least one protein belonging to the bHLH family, chosen among NGN2, ASCL1 and NEUROD1, or fragment thereof, and/or
- at least a nucleic acid molecule coding for said bHLH proteins, i.e. coding for NGN2, ASCL1 and NEUROD1 proteins, or fragment thereof.

An advantageous embodiment discloses herein also relates to a method as defined above, wherein
said NGN2 protein comprises or consists of
- the amino acid sequence SEQ ID NO:1, or
- any amino acid sequence having at least 85% of identity with the amino acid sequence SEQ ID NO :1, preferably any amino acid sequence having at least 90% of identity with the amino acid sequence SEQ ID NO:1, or
- a fragment of said amino acid molecules provided that said fragments induce cell death, in particular apoptosis, preferably said fragments comprising or being constituted by the amino acid sequence SEQ ID NO: 5,
   and/or
   said ASCL1 protein comprises or consists of
- the amino acid sequence SEQ ID NO:2, or
- any amino acid sequence having at least 85% of identity with the amino acid sequence SEQ ID NO :2, preferably any amino acid sequence having at least 90% of identity with the amino acid sequence SEQ ID NO:2,
- a fragment of said amino acid molecules provided that said fragments induce cell death, in particular apoptosis, preferably said fragments comprising or being constituted by the amino acid sequence SEQ ID NO: 6,
   and/or
   said NEUROD1 protein comprises or consists of
- the amino acid sequence SEQ ID NO:9, or
- any amino acid sequence having at least 85% of identity with the amino acid sequence SEQ ID NO :9, preferably any amino acid sequence having at least 90% of identity with the amino acid sequence SEQ ID NO:9, or
- a fragment of said amino acid molecules provided that said fragments induce cell death, in particular apoptosis, preferably said fragments comprising or being constituted by the amino acid sequence SEQ ID NO: 11,
   and/or
   said nucleic acid molecule coding for NGN2 protein comprise or consists of
- the nucleic acid sequence SEQ ID NO : 3, or
- any nucleic acid molecule having at least 75%, preferably at least 85%, more preferably at least 95% of homology with the nucleic acid sequence SEQ ID NO : 3,
- a fragment of nucleic acid molecules provided that said fragments induce cell death, in particular apoptosis, preferably said fragments comprising or being constituted by the amino acid sequence SEQ ID NO 7, coding for the fragment comprising or being constituted by the amino acid sequence SEQ ID NO : 5 as defined above,
   and/or
   said nucleic acid molecule coding for ASCL1 protein comprise or consists of
- the nucleic acid sequence SEQ ID NO : 4, or
- any nucleic acid molecule having at least 75%, preferably at least 85%, more preferably at least 95% of homology with the nucleic acid sequence SEQ ID NO : 2,
- a fragment of said nucleic acid molecules provided that said fragments induce cell death, in particular apoptosis, preferably said fragments comprising or being constituted by the nucleic acid sequence SEQ ID NO 8, coding for the fragment comprising the amino acid sequence SEQ ID NO : 6 as defined above,
   and/or
   said nucleic acid molecule coding for NEUROD1 protein comprise or consists of
- the nucleic acid sequence SEQ ID NO : 10, or
- any nucleic acid molecule having at least 75%, preferably at least 85%, more preferably at least 95% of homology with the nucleic acid sequence SEQ ID NO : 10,
- a fragment of nucleic acid molecules provided that said fragments induce cell death, in particular apoptosis, preferably said fragments comprising or being constituted by the amino acid sequence SEQ ID NO 10, coding for the fragment comprising or being constituted by the amino acid sequence SEQ ID NO : 12 as defined above.

The present invention will be illustrated by the following figures and the following examples.

### Figures:

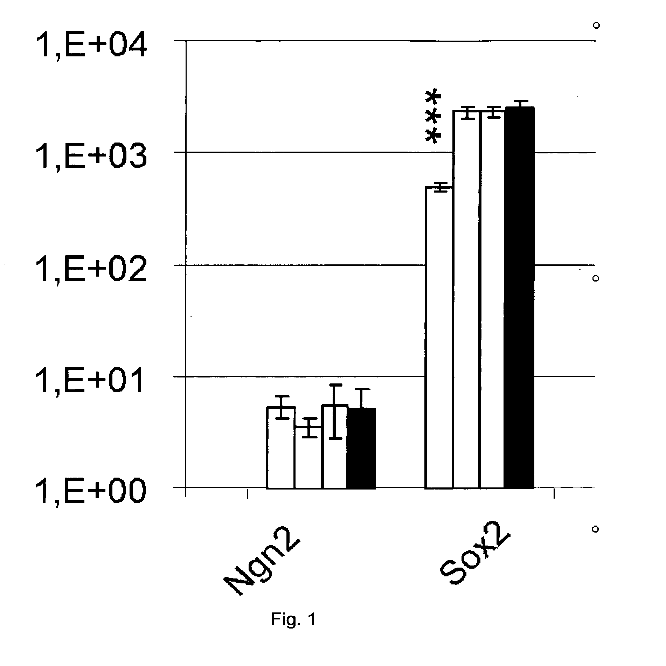

**Figure 1** represents the expression of NGN2 and SOX2 genes, measured by QPCR, in glioma tumors. The columns represent the means of NGN2 and SOX2 expression in 20 tumors of each grade (light grey columns = grade II glioma, dark grey columns = grade III glioma and black columns = grade IV glioma) and 20 non tumoral brains (white columns). Y axis represents arbitrary units on a logarithmic scale of QPCR quantification. This graph indicates that glioma expresses a low level of NGN2 and in contrast a high level of SOX2, a gene typically expressed by undifferentiated stem cells. Non parametric Kruskal and Wallis H tests were used to compare gene expression medians. Error bars represent standard error of mean (sem). Significances: *** (p<0.001).

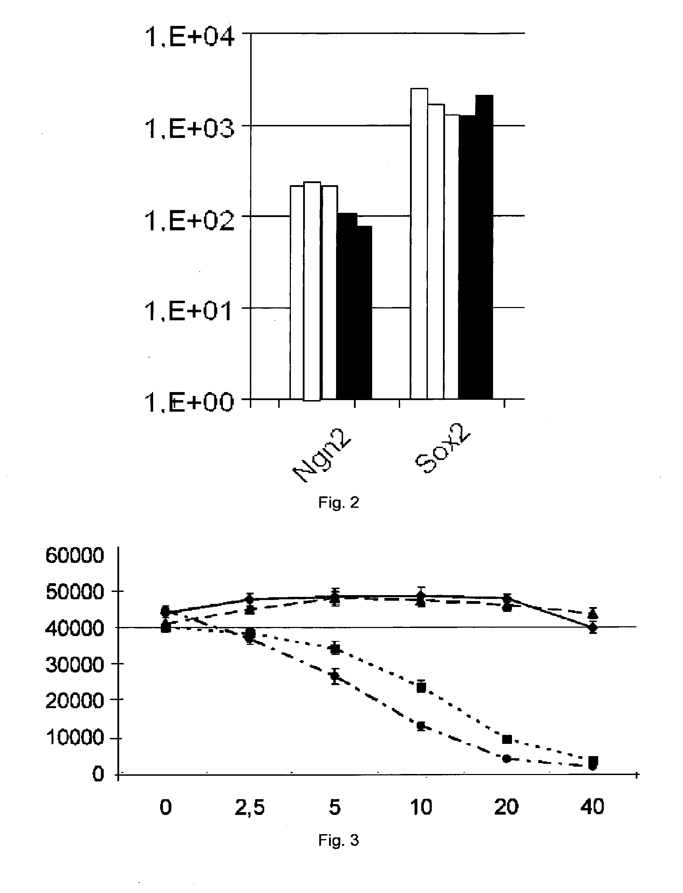

**Figure 2** represents the expression of NGN2 and SOX2 genes, measured by QPCR, in 5 different GBM stem cell primary cultures namely, Gli1 (white columns), Gli2 (light grey columns), Gli3 (middle grey columns), Gli4F11 (dark grey columns) and clonal Gli4F11 (black columns). Y axis represents arbitrary units on a logarithmic scale of QPCR quantification. This graph indicates that GBM stem cells express a low level of NGN2 and in contrast a higher level of SOX2, a gene typically expressed by undifferentiated stem cells.
**Figure 3** represents the number of cells remaining one week after infection of GBM stem cells (Gli4F11) cultured as neurospheres, with increasing amount of integrative or non integrative *NGN2* or GFPexpressing lentivirus. This graph indicates that either integrative or non integrative NGN2 lentiviruses eliminate the vast majority of GBM stem cells. X- axis represents ng of p24 protein per 10⁴ cells and Y-axis the number of cell per well.Curve with black lozenges represents cells infected with integrative lentivirus expressing GFP, curve with black triangles represents cells infected with non integrative lentivirus expressing GFP, curve with black circles represents cells infected with integrative lentivirus expressing NGN2 and curve with black squares represents cells infected with non integrative lentivirus expressing NGN2.

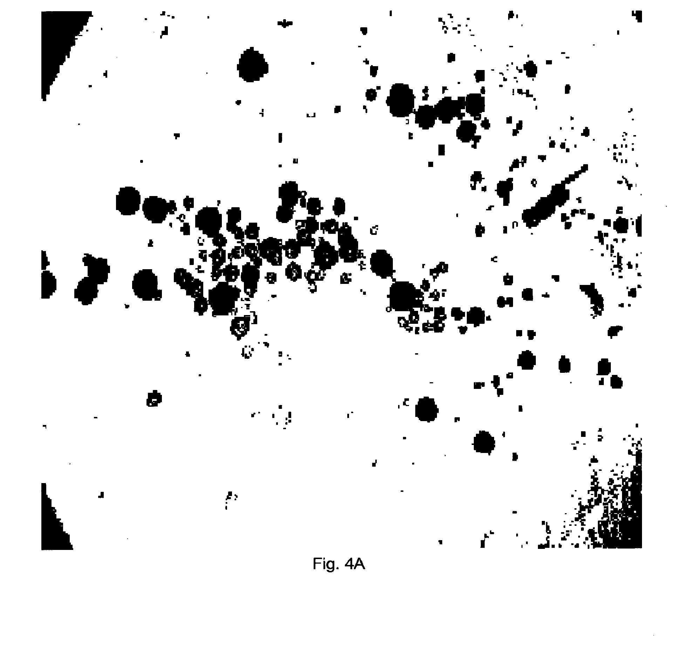

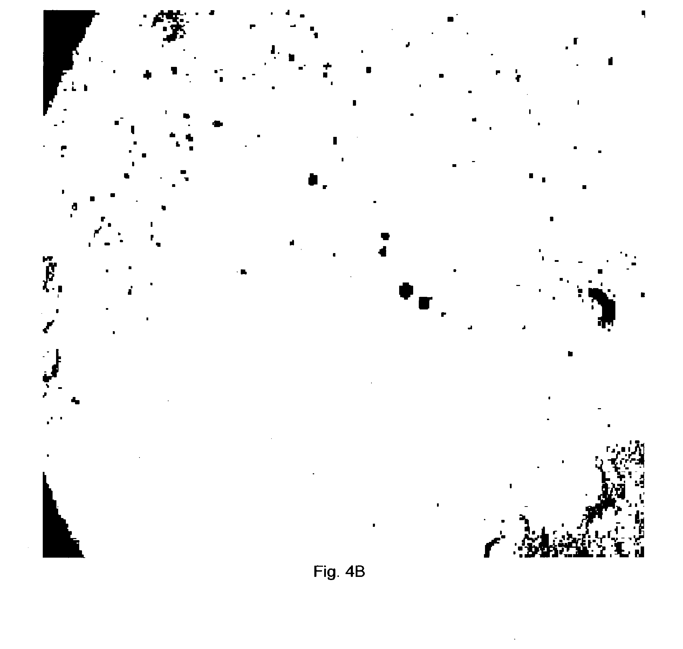

**Figures 4A** **and** **4B** represent photographs of GBM stem cells (Gli4F11) cultured as neurospheres infected with lentivirus, 7 days after infection. Scale bar= 200 µm.
Figure 4A represents neurospheres obtained from cells infected with control lentivirus (GFP)
Figure 4B represents neurospheres obtained from cells infected with lentivirus allowing the expression of NGN2.

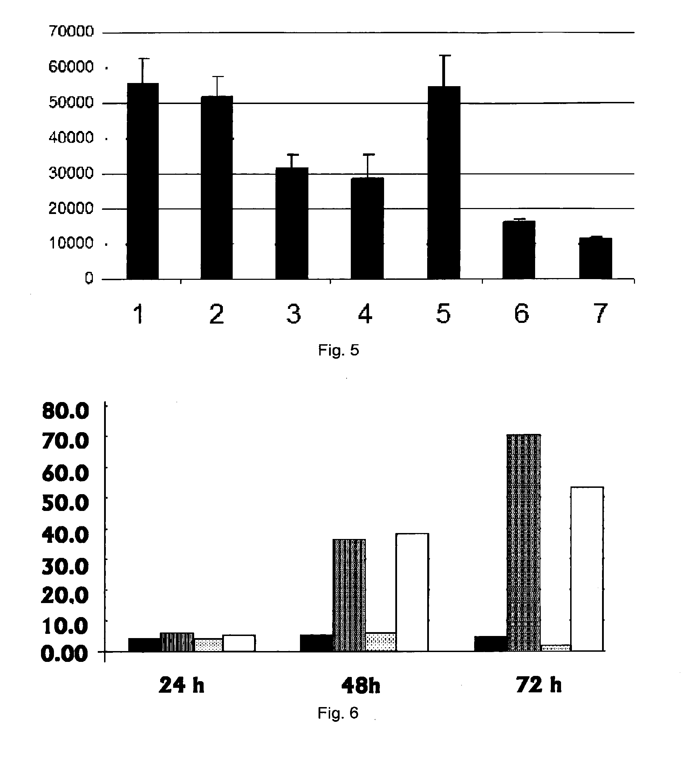

**Figure 5** corresponds to a graph representing the effect of NGN2 or ASCL1 lentivirus infection on GBM stem cell number. First column represents the number of cells when non infected. Column 2 represents the number of cells when infected with integrative lentivirus expressing GFP. Column 3 represents the number of cells when infected with integrative lentivirus expressing NGN2. Column 4 represents the number of cells when infected with integrative lentivirus expressing ASCL1. Column 5 represents the number of cells when infected with non integrative lentivirus expressing GFP. Column 6 represents the number of cells when infected with non integrative lentivirus expressing NGN2. Column 7 represents the number of cells when infected with a non integrative lentivirus expressing ASCL1. These results were obtained using 100 ng of p24 proteins per 10⁵ cells.
**Figure 6** represents the percentage of apoptotic cells (TUNEL positive cells) 24, 48 or 72h after viral infection using 100ng of p24 protein per 10⁵ cells. In each group, first column (black column) represents the number of TUNEL positive cells infected by integrative GFP, second column (dark grey column) represents the number of TUNEL positive cells infected by integrative NGN2, third column (light grey column) represents the number of TUNEL positive cells infected by non integrative GFP and fourth column (white column) represents the number of TUNEL positive cells infected by non integrative NGN2.
**Figure 7** corresponds to a graph representing the percentage of proliferating cells, as measured by KI67 immunofluorescence, after infection with integrative lentivirus (using 100ng of p24 protein per 10⁵ cells) expressing *GFP(*column 1) or expressing *NGN2*(column 2).
**Figure 8** illustrates the predominant action of NGN2 over that of NGN1 and NGN3. The graph represents the number of GBM stem cells remaining 20 days after the infection of integrative lentiviruses (using 100ng of p24 protein per 10⁵ cells). Column 1 represents cells infected with GFP-expressing lentivirus; column 2 represents cells infected with NGN1-expressing lentivirus, column 3 represents cells infected with NGN2-expressing lentivirus and column 4 represents cells infected with NGN3-expressing lentivirus.
**Figures 9** **A and B** represent photographs of remaining GBM stem cells (Gli4F11), grown on an adherent substrate (laminin), 10 days after infection.
Figure 9A represents cells infected with GFP-expressing lentivirus.
Figure 9B represents cells infected with NGN2-expressing lentivirus. NGN2 cells have a typical neuronal morphology.
**Figure 10** represents the detection, by immunofluorescence, of the expression of the typical neuronal protein Doublecortin (Dcx) in NGN2 infected cells (white cytoplasmic staining).
**Figures 11**A and B illustrate the electrophysiological analysis of GFP and NGN2 infected cells.
Figure 11A shows a large inward current (arrow) detected in NGN2 infected cells, but not in control GFP infected cells.
Figure 11B represents action potential recordings from the same cells. Only NGN2 infected cells were able to trigger an action potential (arrow) (mean depolarizing current was 0.45 ± 0.10 nA, n = 4). Increasing the amplitude of depolarizing current to 1.5-2 nA failed to elicit electrical activity in GFP infected cells.
**Figure 12** represents the percentage of cells from Gli4, Gli5, Gli7 polyclonal lines remaining 7 days after lentiviral infection using 100ng of p24 protein per 10⁵ cells. In each group, first column (dark grey column) represents the number of cells infected by integrative GFP, second column (light grey column) represents the cells infected by integrative NGN2. Y axis represents the percentage of cells. Stars indicate signifant Mann-Whitney test (p<0,01).

### Examples:

### Example 1

### EFFECT OF NGN2 OR ASCL1 OR NEUROD1 EXPRESSION ON TUMORAL CELL DEATH.

### Material and methods.

### Cellular Model

The effect of surexpressing NGN2, ASCL1 and NEUROD1 cDNAs in GBM stem cells was investigated using four cancer stem cell lines (Gli4F11, Gli4, Gli5, Gli7) which was derived in the lab from patients diagnosed with a high grade glioma tumor (grade IV according to WHO classification).

These lines grow and self-renew in non adherent conditions and are able to form clonal neurospheres when seeded at one cell in 96 wells dish.

These lines have an abnormal caryotype.

These lines express typical cancer stem cell markers (nestin, CD133, CD15) and contain a side population (Hoescht exclusion).

These lines are multipotential and generate GFAP+, Map2ab+ and GalC+ cells after differentiation.

These line generate highly infiltrating high grade tumors in NOD/SCID mice. After 4 months, the whole brain is invaded by tumoral cells which remain proliferative.

### Cell Culture

Gli4F11, Gli4, Gli5, Gli7 cells are cultured in serum-free DMEM/F12 media supplemented with non vitamin A-B27 (2%) and N2 (1%) serum-replacement media (Invitrogen), FGF2, EGF2 (10 ng/ml, Peproteck,), Heparine (2 µg/ml, Sigma), Ciprofloxaxine (2 µg/ml, Euromedex), Gentamycine (10 µg/ml, Fisher), fungin (10 µg/ml, Cayla) and fungizone (0,25 µg/ml, Fisher). The cell are passaged classically using complete dissociation with trypsine/EDTA 0, 25% for 3' followed by trypsin inactivation with trypsin inhibitor (Sigma, T9003). Cells are either grown free-floating on non-adherent substrate coated flasks (poly-2-hydroxyethylmethacrylate, poly HEMA from Sigma) or adherent substrate coated dishes (poly-D-lysine (25 µg/ml) and laminin (2 µg/cm2, Sigma)).

### Lentivirus construction

The human NGN2, ASCL1 and NEUROD1 cDNAs were cloned between the house keeping promoter of the phosphoglycerate kinase gene (PGK) and the Woodchuck hepatitis virus post-transcriptional regulating élément (WPRE) in the plasmid Trip-PGK-WPRE coding for a self inactivating HIV-1 derived vector containing a central cPPT sequence (Zennou V, et al. (2001) Nat Biotechnol 19(5):446-450). The inventors produced two types of HIV-1 derived vectors, either carrying a wild type or a mutant (D64V) integrase, leading respectively to integrating (Trip-PGK-Ngn2) and non-integrating (Ni-Trip-PGK-ngn2) vector phenotypes (Sarkis C, et al. (2008) Curr Gene Ther8(6):430-437). Stocks of vectors were prepared by transitory transfection of 293T cells with the p8.91 encapsidation plasmid ( ΔVprΔVifΔVpuΔNef) (Zufferey R, et al. (1997) Nat Biotechnol 15(9):871-875) either with a wt or a mutated integrase, pHCMV-G encoding the VSV envelope and a plasmid containing the vector, as described previously (Zennou V, et al. (2001) Nat Biotechnol 19(5):446-450). Briefly, the supernatants were treated with DNAse I prior to ultracentrifugation then resuspended in PBS and frozen (-80°) until use. The amount of p24 capsid protein was determined by HIV-1 core profile ELISA (NEN) as described by the manufacturer.

### RESULTS

### NGN2 expression in glioma (figure 1 and 2)

Figure 1 and 2 illustrate that *NGN2* is weakly expressed in glioma tumors (grade II to IV) and in five GBM stem cell cultures compared to *SOX2,* a gene important for maintaining GBM stem cell undifferentiated. This shows the predominance of an undifferentiatied state in glioma and GBM stem cells.

### Effect of lentiviral infection on cell number (figure 3, 4, 5)

Non integrating or integrating lentiviruses expressing NGN2, ASCL1 or GFP (control) were used to infect GIi4F11 GBM cells growing as neurospheres (non adherent condition).

First, cells dissociated with trypsin were seeded at 10 000 cells per well in poly HEMA-coated 24 wells plate containing 1 ml of growth media and lentiviral particles (between 0 to 40 ng of p24 protein per well, 4 wells per conditions). After seven days, the cell number was determined by direct trypsinization of the cells by adding 300 µl of Trypsin 2,5% (sigma, 4799) into the well. After 10 minutes at 37°C, the cells were triturated and their number was counted using Z2 coulter counter (Beckman) using a 10-20 µm window.

Results are shown in figures 3, 4A and B and 5.

Figure 3 shows that compared to control GFP lentiviruses, NGN2 integrative or non integrative lentiviruses induce a massive reduction of the GBM stem cells number.

Figure 4A and B illustrates the aspect of cultures, 7 days after treatment with GFP or NGN2 lentiviruses. Note the almost complete disappearance of neurospheres on the right photograph (B).

Figure 5 shows that ASCL1 is as efficient as NGN2 as reducing the cell number. Compared to GFP-lentivirus treated cells (column 2 for integrative virus and 5 for non integrative virus), both NGN2 (column 3 for integrative virus and 6 for non integrative virus) and ASCL1 (column 4 for integrative virus and 7 for non integrative virus) dramatically reduced the cell number.

### Determination of the rate of apoptosis induced by the lentiviral infection (figure 6)

To assess the rate of apoptosis induced by the lentiviral infection, the cells were seeded at 150 000 cells per wells in a 24-wells plate (containing poly D-Lysine/Laminin coated coverslips) with 1 ml of media and lentiviral particles (150 ng of p24 protein). At 24h, 48 and 72h after the infection, the cells were fixed with an ethanol:acetic acid 2:1 (v:v) solution. Apoptotic cells were detected using the TUNEL method with an Apoptag red in situ apotosis detection kit (Millipore) according to the manufacturer's protocol.

Results are shown on figure 6.

48 hours post infection, a massive apoptosis (up to 40 % of cells) is detected in cells infected with integrative or non integrative NGN2 lentiviruses compared to cells infected with GFP lentiviruses. The rate of apoptosis is further increased at 72 h post infection to reach 50-60 % of the cells.

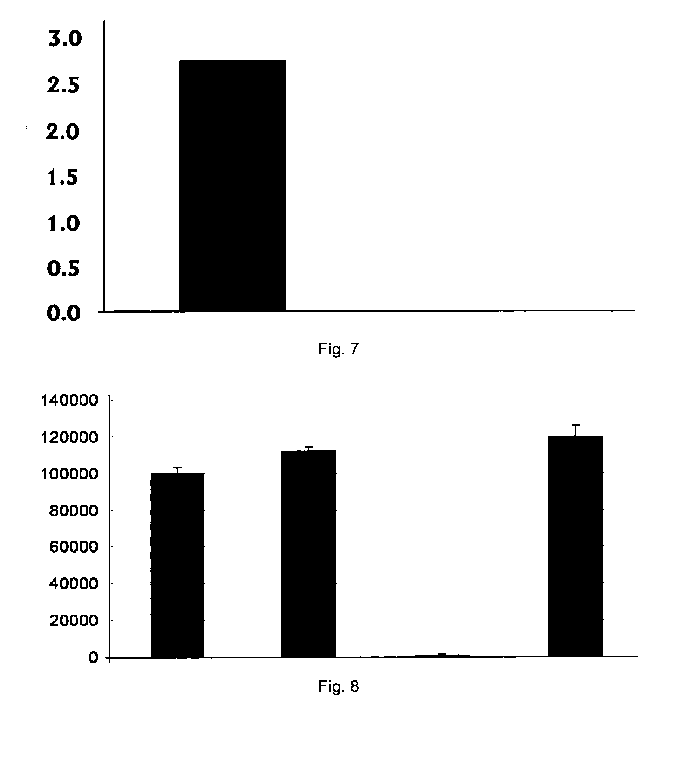

### Determination of the proliferation inhibition induced by the lentiviral infection (figure 7)

Cells were seeded with the same conditions as those used for apoptosis detection. After 7 days, the cells were fixed with 4% paraformaldehyde and the rate of proliferation was measured using detection of the nuclear KI67 (monoclonal antibody BD) antigen by classical immunofluorescence.

Results are shown on figure 7. Compared to cells infected with a GFP integrative lentivirus, virtually no dividing KI67⁺ cells can be detected in NGN2 lentivirus treated cells.

### Specific effect of NGN2 over NGN1 and NGN3 lentiviruses (figure 8)

GBM stem cells were infected with the same amount of p24 protein of NGN1, NGN2 and NGN3 integrative lentiviruses, then the cell number was determined after 20 days. Figure 8 shows that NGN1 and NGN3 were not able to reduce the cell number compared to NGN2 lentiviruses. This illustrates the specific effect of the NGN2 sequence to induce GBM stem cell reduction.

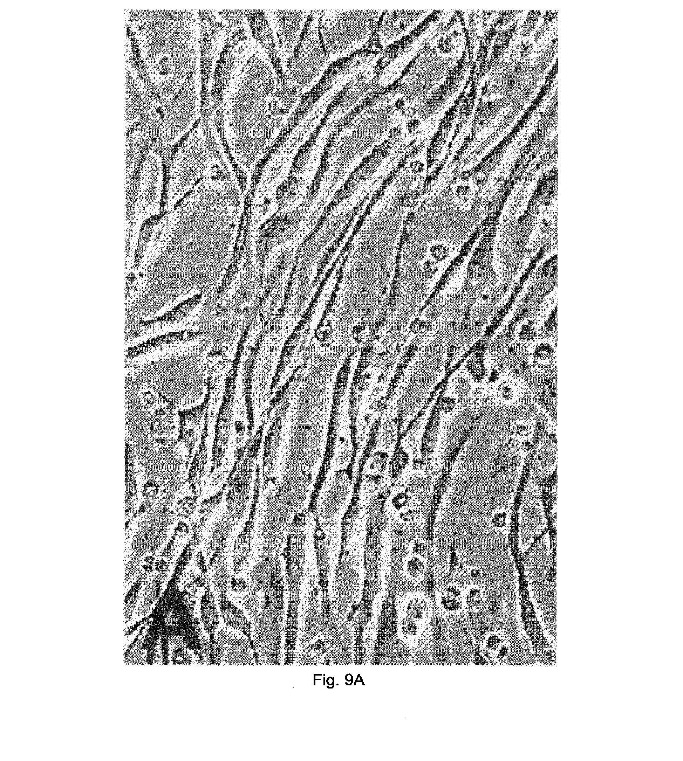

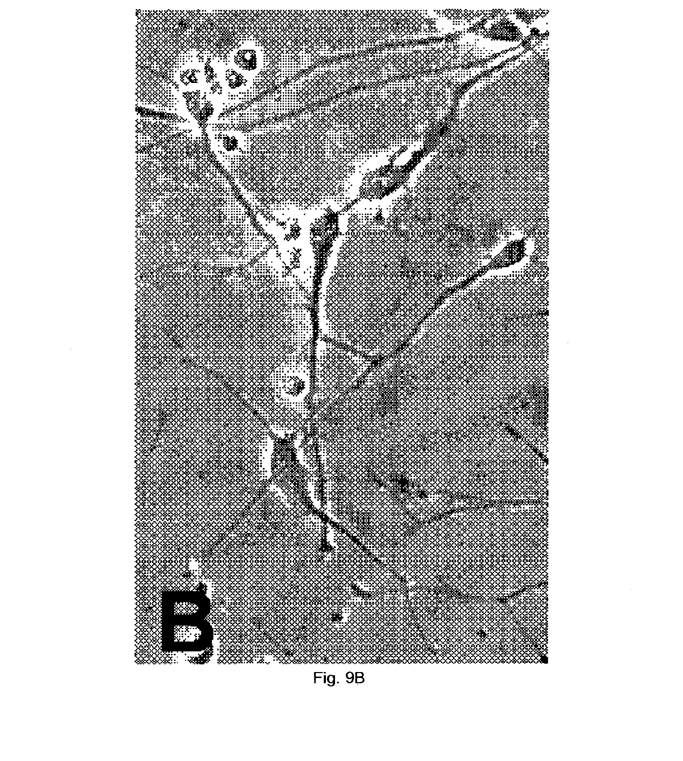

### Forced expression of NGN2 in GBM stem cells induces neuronal differentiation (figure 9, 10, 11)

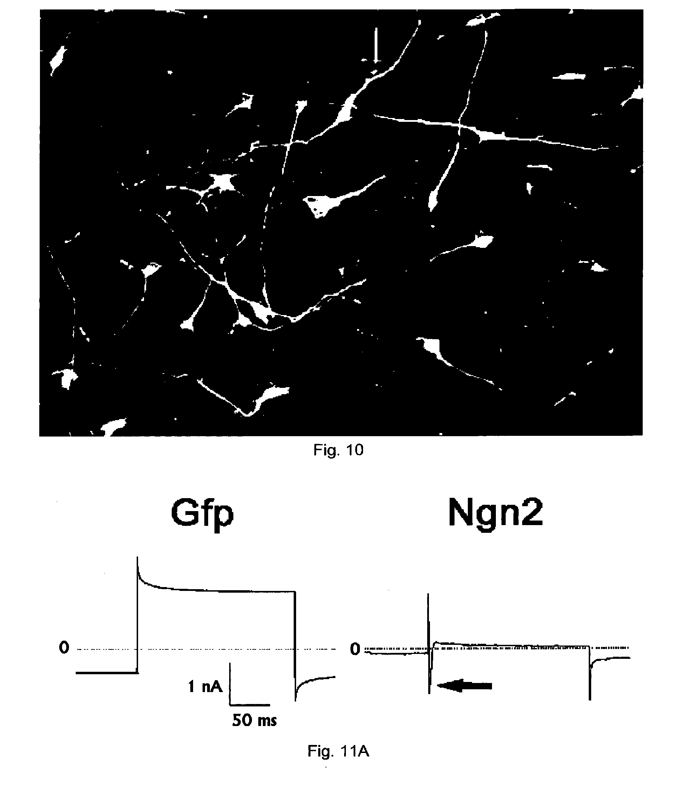

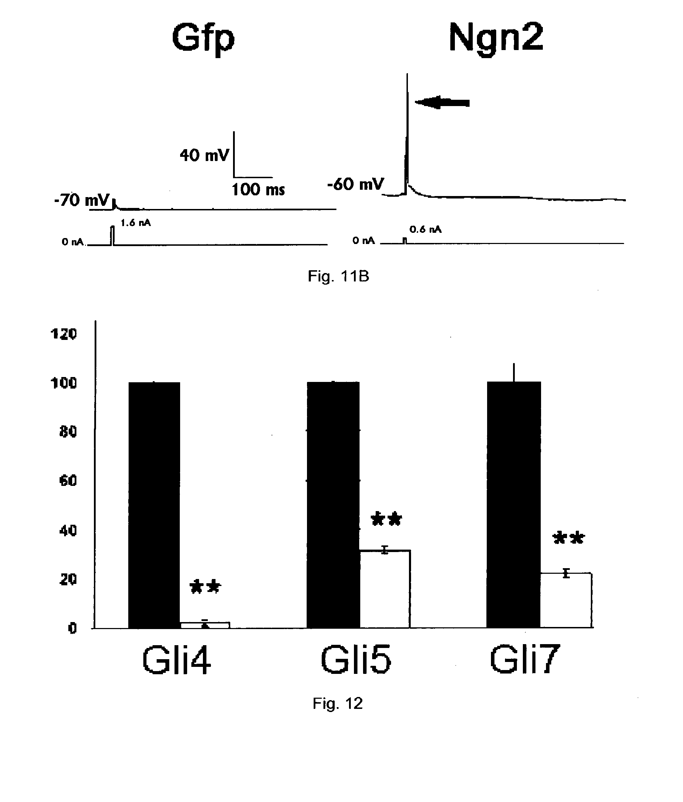

In addition to cell death, NGN2 lentivirus infection induces a striking cell morphology change. This is illustrated on figure 9 which shows that cells infected with GFP lentiviruses display a bipolar fusiform morphology (A) while those infected with NGN2 lentiviruses have a typical neuronal morphology (B). The neuronal phenotype of NGN2 infected cells is also demonstrated by the high expression of the neuronal specific marker Doublecortin (Dcx) detected by immunofluorescence (white cytoplasmic staining on figure 10). This neuronal phenotype is also supported by electrophysiology analysis which shows that a large inward current (arrow) was detected in 6 out of 7 recorded cells infected with NGN2 lentiviruses (mean peak amplitude was -515 ± 125 pA) whereas control cells infected with GFP lentiviruses only elicited outward currents (figure 11 A). In addition, figure 11B shows that only NGN2 lentivirus infected cells were able to trigger an action potential (arrow) (mean depolarizing current was 0.45 ± 0.10 nA, n = 4). Increasing the amplitude of depolarizing current to 1.5-2 nA failed to elicit electrical activity in GFP lentivirus infected cells.

For NeuroD1, all the results obtained with this lentivirus are similar to those obtained with NGN2 and ASCL1 viruses

### Example 2 : Inhibition of polyclonal glioma cell growth by Ngn2 overexpression

In addition to the clonal Gli4F11 cell line, the effect of overexpressing Ngn2 was extended in 3 others glioma cultures.

The Inventors confirmed their results by using the primary polyclonal Gli4 culture from which Gli4F11 was derived and by deriving two new cultures (Gli5 and Gli7) from two patients affected by glioblastoma. Like Gli4F11, these three lines present a phenotype reminiscent of proneural cells as evidenced by the expression of Ascl1, NG2/CSPG4, Nkx2.2, Olig 1/2 and PDGFRα markers. These cultures are multipotent, have abnormal karyotypes and form highly infiltrative Olig2⁺ Nkx2.2⁺ tumors similar to Gli4F11 when grafted in immunocompromised mice. In these 3 cultures, overexpression of Ngn2 using lentivirus, drastically reduced the cell number compared to control condition (GFP) after 5 days of culture, which was associated with an overt cell death, as shown in **figure 12****.**

Similar results were obtained in Gli5 and Gli7 cells by using ASCL1 and NEUROD1 lentivirus.

## Claims

1. Composition comprising:
- at least one protein belonging to the bHLH family, chosen among NGN2, ASCL1, and NEUROD1 and/or
- at least a nucleic acid molecule coding for said bHLH proteins,
for its use for the treatment of central nervous system (CNS) tumors and neuroendocrine tumors.

2. Composition for its use according to claim 1, wherein said NGN2 protein comprises or consists of:
- the amino acid sequence SEQ ID NO:1, or
- any amino acid sequence having at least 85% of identity with the amino acid sequence SEQ ID NO :1, preferably any amino acid sequence having at least 90% of identity with the amino acid sequence SEQ ID NO:1.

3. Composition for its use according to claim 1, wherein said ASCL1 protein comprises or consists of:
- the amino acid sequence SEQ ID NO:2, or
- any amino acid sequence having at least 85% of identity with the amino acid sequence SEQ ID NO :2, preferably any amino acid sequence having at least 90% of identity with the amino acid sequence SEQ ID NO:2.

4. Composition for its use according to claim 1, wherein said NEUROD1 protein comprises or consists of:
- the amino acid sequence SEQ ID NO:9, or
- any amino acid sequence having at least 85% of identity with the amino acid sequence SEQ ID NO :9, preferably any amino acid sequence having at least 90% of identity with the amino acid sequence SEQ ID NO:9.

5. Composition for its use according to claim 1, wherein said nucleic acid molecule coding for NGN2 protein comprise or consists of:
- the nucleic acid sequence SEQ ID NO : 3, or
- any nucleic acid molecule having at least 75%, preferably at least 85%, more preferably at least 95% of homology with the nucleic acid sequence SEQ ID NO : 3.

6. Composition for its use according to claim 1, wherein said nucleic acid molecule coding for ASCL1 protein comprise or consists of:
- the nucleic acid sequence SEQ ID NO : 4, or
- any nucleic acid molecule having at least 75%, preferably at least 85%, more preferably at least 95% of homology with the nucleic acid sequence SEQ ID NO : 4.

7. Composition for its use according to claim 1, wherein said nucleic acid molecule coding for NEUROD1 protein comprise or consists of:
- the nucleic acid sequence SEQ ID NO : 10, or
- any nucleic acid molecule having at least 75%, preferably at least 85%, more preferably at least 95% of homology with the nucleic acid sequence SEQ ID NO : 10.

8. Composition for its use according to anyone of claims 1 or 5 to 7, wherein each of said at least nucleic acid molecule is comprised in one vector, said vector comprising nucleic acid sequences allowing the expression of said nucleic acid molecule.

9. Composition for its use according to claim 1 or 5 to 7, wherein
- a first nucleic acid molecule coding for said NGN2 protein,
- a second nucleic acid molecule coding for said ASCL1 protein, and
- a third nucleic acid molecule coding for said NEUROD1 protein,
are comprised in the same vector,
said first, second and third nucleic acid molecules being are placed under the control of nucleic acid sequences allowing the expression of said nucleic acid molecules.

10. Composition for its use according to anyone of claims 1 to 8, wherein said composition comprises:
- either at least two protein belonging to the bHLH family, chosen among NGN2, ASCL1, and NEUROD1,
- or at least two nucleic acid molecule coding for said bHLH proteins,
- or at least one protein belonging to the bHLH family, chosen among NGN2, ASCL1, and NEUROD1, and at least a nucleic acid molecule coding for said bHLH proteins,
which are used in a simultaneous, separate or sequential manner.

11. Composition for its use according to anyone of claims 1 to 10, further comprising at least one antitumoral agent.

12. Composition for its use according to claim 11, wherein :
- at least one protein belonging to the bHLH family, chosen among NGN2, ASCL1, and NEUROD1, and/or
- at least a nucleic acid molecule coding for said bHLH proteins,
and
said at least one antitumoral agent
are used in a simultaneous, separate or sequential manner.

13. Composition for its use according to anyone of claims 1 to 12, wherein said CNS tumors are chosen among the group consisting of grade II-IV glioma according to the 2007 WHO classification of tumours of the central nervous system.

14. Composition for its use according to anyone of claims 1 to 12, wherein said neuroendocrines tumors are chosen among the group consisting of primary or metastatic Gastro-entero-pancreatic neuroendocrine tumors.

## Patentansprüche

1. Zusammensetzung die:
- mindestens ein Protein aus der bHLH-Familie gewählt unter NGN2, ASCL1 und NEUROD1 und / oder
- zumindest ein Nukleinsäuremolekül, das das bHLH-Protein für ihre Verwendung zur Behandlung zentralen Nervensystems (ZNS) und neuroendokrinen Tumoren kodiert,
umfasst.

2. Zusammensetzung für ihre Verwendung nach Anspruch 1, wobei das NGN2-Protein:
- die Aminosäuresequenz SEQ ID NO: 1, oder
- jede Aminosäuresequenz, die mindestens 85% der Identität der Aminosäuresequenz SEQ ID NO: 1 teilt, vorzugsweise jede Aminosäuresequenz, die wenigstens 90% der Identität der Aminosäuresequenz SEQ ID NO: 1 teilt,
umfasst oder daraus besteht.

3. Zusammensetzung für ihre Verwendung nach Anspruch 1, wobei das ASCL1-Protein:
- die Aminosäuresequenz SEQ ID NO:2, oder
- jede Aminosäuresequenz, die mindestens 85% der Identität der Aminosäuresequenz SEQ ID NO:2, vorzugsweise jede Aminosäuresequenz, die wenigstens 90% der Identität der Aminosäuresequenz SEQ ID NO:2 teilt,
umfasst oder daraus besteht.

4. Zusammensetzung für ihre Verwendung nach Anspruch 1, wobei das NEUROD1-Protein :
- die Aminosäuresequenz SEQ ID NO:9, oder
- jede Aminosäuresequenz, die mindestens 85% der Identität der Aminosäuresequenz SEQ ID NO:9, vorzugsweise jede Aminosäuresequenz, die wenigstens 90% der Identität der Aminosäuresequenz SEQ ID NO:9 teilt,
umfasst oder daraus besteht.

5. Zusammensetzung für ihre Verwendung nach Anspruch 1, wobei das NGN2-Protein kodierende Nukleinsäuremolekül:
- die Nukleinsäuresequenz SEQ ID NO: 3, oder
- jedes Nukleinsäuremolekül, das mindestens 75%, vorzugsweise mindestens 85%, am liebsten mindestens 95% Homologie mit der Nukleinsäuresequenz SEQ ID NO: 3 zeigt,
umfasst oder daraus besteht.

6. Zusammensetzung für ihre Verwendung nach Anspruch 1, wobei das ASCL1-Protein kodierende Nukleinsäurenmolekül:
- die Nukleinsäuresequenz SEQ ID NO: 4, oder
- jedes Nukleinsäuremolekül, das mindestens 75%, vorzugsweise mindestens 85%, am liebsten mindestens 95% Homologie mit der Nukleinsäuresequenz SEQ ID NO: 4 zeigt,
umfasst oder daraus besteht.

7. Zusammensetzung für ihre Verwendung nach Anspruch 1, wobei das NEUROD1-Protein kodierenden Nukleinsäuremolekül:
- die Nukleinsäuresequenz SEQ ID NO: 10, oder
- jedes Nukleinsäuremolekül, das mindestens 75%, vorzugsweise mindestens 85%, am liebsten mindestens 95% Homologie mit der Nukleinsäuresequenz SEQ ID NO: 10 zeigt,
umfasst oder daraus besteht.

8. Zusammensetzung für ihre Verwendung nach einem der Ansprüche 1 oder 5 bis 7, wobei jedes der mindestens ein Nukleinsäuremolekül in einem Träger enthalten ist, wobei der Träger Nukleinsäuresequenzen, die die Expression des Nukleinsäuremoleküls ermöglichen, umfasst.

9. Zusammensetzung für ihre Verwendung nach einem der Ansprüche 1 oder 5 bis 7, wobei
- ein erstes Nukleinsäuremolekül für das NGN2-Protein kodiert,
- ein zweites Nukleinsäuremolekül für das ASCL1-Protein kodiert,
- ein drittes Nukleinsäuremolekül für das NEUSOD1-Protein kodiert,
in dem gleichen Träger enthalten sind,
wobei das erste, zweite und dritte Nukleinsäuremolekül unter der Kontrolle von Nukleinsäuresequenzen, die die Expression des Nukleinsäuremoleküls ermöglichen, gebracht werden.

10. Zusammensetzung für ihre Verwendung nach einem der Ansprüche 1 bis 8, wobei die Zusammensetzung:
- entweder mindestens zwei an der bHLH-Familie gehörende Proteine, gewählt unter NGN2, ASCL1 und NEUROD1,
- oder mindestens zwei für das bHLH-Proteine kodierende Nucleinsäuremoleküle,
- oder mindestens ein der bHLH-Familie gehörendes Protein, gewählt unter NGN2, ASCL1 und NEUROD1 und mindestens ein für das bHLH-Proteine kodierendes Nucleinsäuremolekül, die in einer gleichzeitigen, getrennten oder sequentiellen Weise verwendet werden,
umfasst.

11. Zusammensetzung für ihre Verwendung nach einem der Ansprüche 1 bis 10, weiterhin mindestens ein Antitumormittel umfassend.

12. Zusammensetzung für ihre Verwendung nach Anspruch 11 in der:
- mindestens ein an der bHLH-Familie gehörendes Protein, gewählt unter NGN2, ASCL1 und NEUROD1, und/oder
- mindestens ein für bHLH-Proteine kodierende Nucleinsäuremolekül,
und
das mindestens eine Antitumormittel in einer gleichzeitigen, getrennten oder sequentiellen Weise verwendet werden.

13. Zusammensetzung für die Verwendung nach einem der Ansprüche 1 bis 12, wobei die ZNS-Tumoren aus der Gruppe, die aus, der 2007 WHO Tumoren des Zentralnervensystems Klassifikation nach, Grad II-IV Gliomen besteht, ausgewählt werden.

14. Zusammensetzung für ihre Verwendung nach einem der Ansprüche 1 bis 12, wobei die neuroendocrinen Tumoren aus der Gruppe, die aus primären oder metastatischen Gastro-entero-neuroendokriner Tumoren bestehet, ausgewählt werden.

## Revendications

1. Composition comprenant:
- au moins une protéine appartenant à la famille bHLH, choisie parmi NGN2, ASCL1, et NEUROD1 et/ou
- au moins une molécule d'acide nucléique codant lesdites proteins bHLH,
pour son utilisation pour le traitement des tumeurs du système nerveux central (SNC) et des tumeurs neuroendocrines.

2. Composition pour son utilisation selon la revendication 1, où ladite protéine NGN2 comprend ou consiste en :
- la séquence d'acides aminés SEQ ID NO:1, ou
- toute séquence d'acides aminés ayant au moins 85% d'identité avec la séquence d'acides aminés SEQ ID NO : 1, de préférence toute séquence ayant au moins 90% d'identité avec la séquence d'acides aminés SEQ ID NO:1.

3. Composition pour son utilisation selon la revendication 1, où ladite protéine ASCL1 comprend ou consiste en :
- la séquence d'acides aminés SEQ ID NO: 2, ou
- toute séquence d'acides aminés ayant au moins 85% d'identité avec la séquence d'acides aminés SEQ ID NO : 2, de préférence toute séquence ayant au moins 90% d'identité avec la séquence d'acides aminés SEQ ID NO:2.

4. Composition pour son utilisation selon la revendication 1, où ladite protéine NEUROD1 comprend ou consiste en :
- la séquence d'acides aminés SEQ ID NO:9, ou
- toute séquence d'acides aminés ayant au moins 85% d'identité avec la séquence d'acides aminés SEQ ID NO : 9, de préférence toute séquence ayant au moins 90% d'identité avec la séquence d'acides aminés SEQ ID NO:9.

5. Composition pour son utilisation selon la revendication 1, où ladite molécule d'acide nucléique codant NGN2 comprend ou consiste en :
- la séquence d'acide nucléique SEQ ID NO : 3, ou
- toute séquence d'acide nucléique ayant au moins 75% d'identité, de préférence au moins 85% d'identité, plus préférentiellement au moins 95% d'identité avec la séquence d'acide nucléique SEQ ID NO : 3.

6. Composition pour son utilisation selon la revendication 1, où ladite molécule d'acide nucléique codant ASCL1 comprend ou consiste en :
- la séquence d'acide nucléique SEQ ID NO : 4, ou
- toute séquence d'acide nucléique ayant au moins 75% d'identité, de préférence au moins 85% d'identité, plus préférentiellement au moins 95% d'identité avec la séquence d'acide nucléique SEQ ID NO:4.

7. Composition pour son utilisation selon la revendication 1, où ladite molécule d'acide nucléique codant for NEUROD1 comprend ou consiste en :
- la séquence d'acide nucléique SEQ ID NO : 10, or
- toute séquence d'acide nucléique ayant au moins 75% d'identité, de préférence au moins 85% d'identité, plus préférentiellement au moins 95% d'identité avec la séquence d'acide nucléique SEQ ID NO : 10.

8. Composition pour son utilisation selon l'une quelconque des revendications 1 ou 5 à 7, ou chacune desdites molécules d'acide nucléique est comprise dans un vecteur, ledit vecteur comprenant des séquences d'acide nucléique permettant l'expression desdites molécules d'acide nucléique.

9. Composition pour son utilisation selon la revendication 1 ou 5 à 7, ou
- une première molécule d'acide nucléique codant ladite protéine NGN2,
- une seconde molécule d'acide nucléique codant ladite protéine ASCL1, et
- une troisième molécule d'acide nucléique codant ladite protéine NEUROD1, sont comprises dans le même vecteur,
lesdites première, seconde et troisième molécules d'acide nucléique étant placées sous contrôle de séquences d'acide nucléique permettant l'expression desdites molécules d'acide nucléique.

10. Composition pour son utilisation selon l'une quelconque des revendications for its 1 à 8, où ladite composition comprend:
- soit au moins deux protéines appartenant à la famille bHLH family, choisies parmi NGN2, ASCL1, et NEUROD1,
- ou au moins deux molécules d'acide nucléique codant lesdites protéines bHLH,
- ou au moins une protéine appartenant à la famille bHLH, choisie parmi NGN2, ASCL1, et NEUROD1, et au moins une molécule d'acide nucléique codant lesdites protéines bHLH,
qui sont utilisées d'une manière simultanée, séparée ou séquentielle.

11. Composition pour son utilisation selon l'une quelconque des revendications 1 à 10, comprenant en outre au moins un agent antitumoral.

12. Composition pour son utilisation selon la revendication 11, où :
- ou au moins une protéine appartenant à la famille bHLH, choisie parmi NGN2, ASCL1, et NEUROD1, et/ou
- au moins une molécule d'acide nucléique codant lesdites protéines bHLH,
et
ledit au moins un agent antitumoral
sont utilisées d'une manière simultanée, séparée ou séquentielle.

13. Composition pour son utilisation selon l'une quelconque des revendications 1 à 12, où les dites tumeurs du SNC sont choisies dans le groupe constitué des gliomes de grades II à IV selon la classification de l'OMS en 2007 des tumeurs du système nerveux central.

14. Composition pour son utilisation selon l'une quelconque des revendications 1 à 12, où lesdites tumeurs neuroendocrines t choisies dans le groupe constitué des tumeurs neuroendocrines Gastro-entero-pancreatiques primaires ou métastatiques.
